# EUROPÄISCHE PATENTSCHRIFT

(11) **EP 1 416 982 B1**
(45) Veröffentlichungstag und Bekanntmachung des Hinweises auf die Patenterteilung: **21.03.2007**
(21) Anmeldenummer: 02745036.0
(22) Anmeldetag: 22.07.2002
(51) Int. Cl.: A61M 5/00, A61M 5/315

(54) **Verriegelungssperre für eine Verbindung von Gehäuseabschnitten eines Verabreichungsgeräts**
Locking device for connecting housing sections of an administration appliance
Dispositif de verrouillage destiné à la connexion de sections de bôitier d'un appareil d'administration

(30) Priorität: 30.07.2001 DE 20112501 U; 21.12.2001 DE 10163325
(43) Veröffentlichungstag der Anmeldung: 12.05.2004
(73) Patentinhaber: TecPharma Licensing AG, 3401 Burgdorf (CH)
(72) Erfinder: KIRCHHOFER, Fritz, CH-3454 Sumiswald (CH); GRAF, Roney, CH-3400 Burgdorf (CH)
(74) Vertreter: Schwabe - Sandmair - Marx
(86) Internationale Anmeldenummer: PCT/CH2002/000409
(87) Internationale Veröffentlichungsnummer: WO 2003/011370

(56) Entgegenhaltungen:
- EP-A- 0 498 737
- EP-A- 1 095 668
- WO-A-00/02606
- WO-A-97/17095
- US-A- 5 611 783

## Beschreibung

Die Erfindung betrifft die Verbindung von Gehäuseabschnitten eines Verabreichungsgeräts. Insbesondere ist die Erfindung bei Verabreichungsgeräten von Vorteil, die einen nur einmal verwendbaren Verbrauchsteil und eine für einen mehrmaligen Gebrauch vorgesehene Antriebsvorrichtung oder Dosier- und Antriebsvorrichtung umfassen. Als Semidisposable Pens bezeichnete Injektionsgeräte sind bevorzugte Beispiele hierfür. Die Erfindung kann aber auch für andere Injektionsgeräte und auch für Infusionsgeräte mit Vorteil eingesetzt werden. Ebenso ist sie vorteilhaft für Inhalationsgeräte, um das Produkt für beispielsweise eine Zerstäubung einer Zerstäubungseinrichtung des Inhalationsgeräts aufzugeben.

Injektionsgeräte und auch Infusionsgeräte weisen im Allgemeinen einen Reservoirteil für das zu verabreichende Produkt und eine Antriebsvorrichtung im Falle einer nur einmal möglichen Produktverabreichung oder eine Dosier- und Antriebsvorrichtung im Falle der Auswählbarkeit einer Produktdosis auf. Der Reservoirteil beinhaltet ein Produktreservoir, aus dem durch Vorschub eines Kolbens das Produkt oder ein Teil des Produkts durch einen Reservoirauslass abgegeben wird. Die Antriebsvorrichtung bewirkt den Vorschub des Kolbens.

Falls es sich um ein Gerät für eine mehrmalige dosierte Produktabgabe handelt, ist die Antriebsvorrichtung zu einer Dosier- und Antriebsvorrichtung weiter entwickelt, die auch eine Dosiervorrichtung oder zumindest einen Teil einer Dosiervorrichtung und gegebenenfalls auch eine Anzeigeeinrichtung zum optischen und/oder akustischen Anzeigen der ausgewählten Produktdosis umfasst.

Probleme entstehen bei dem Zusammenbau der Antriebsvorrichtung oder Dosier- und Antriebsvorrichtung und dem Reservoirteil durch den Umstand, dass der Kolben im Reservoir des Reservoirteils mit der Antriebsvorrichtung und gegebenenfalls einer Dosiervorrichtung oder einem Teil einer Dosiervorrichtung gekoppelt werden muss. Probleme können insbesondere entstehen, wenn das Gerät die Auswahl von unterschiedlichen Produktdosen erlaubt, da ein unachtsamer Zusammenbau eines neuen Reservoirteils und einer bereits einmal verwendeten Dosier- und Antriebsvorrichtung die Gefahr einer anfänglichen Fehleinstellung und deshalb einer Fehldosierung für die erste Produktverabreichung nach dem Zusammenbau in sich birgt. Erhöht wird die Gefahr noch, wenn es sich bei dem Injektions- oder Infusionsgerät um ein Gerät für die Selbstverabreichung handelt, wie sie heutzutage in vielen Therapien bereits üblich ist, beispielsweise in der Diabetestherapie.

Aus der EP 1 095 668 A1 ist eine medizinische Verabreichungsvorrichtung bekannt, die ein vorderes und ein hinteres Gehäuseteil umfasst. Diese Gehäuseteile können miteinander lösbar verbunden werden. Das vordere Gehäuseteil weist eine Kartusche mit einem Kolben auf, der auf ein in der Kartusche befindliches Medikament wirkt. Das hintere Gehäuseteil weist ein Betätigungselement, zum Einstellen und Verabreichen einer Medikamentendosis, und eine Kolbenstange auf, die im zusammengesetzten Zustand der Gehäuseteile auf den Kolben wirken kann.

Es ist eine Aufgabe der Erfindung, die Gefahr einer Fehldosierung zu verringern, die aus dem Zusammenbau eines Reservoirteils und einer Antriebsvorrichtung oder Dosier- und Antriebsvorrichtung resultiert.

Ein Verabreichungsgerät nach der Erfindung weist einen vorderen Gehäuseabschnitt auf, der ein Reservoir für das Produkt enthält. In dem Reservoir ist ein Kolben in eine Vorschubrichtung auf einen Auslass des Reservoirs zu verschiebbar aufgenommen, um durch einen Kolbenhub das gesamte Produkt oder vorzugsweise nur eine ausgewählte Produktdosis auszuschütten. Das Verabreichungsgerät, vorzugsweise Injektions- oder Infusionsgerät, umfasst ferner einen hinteren Gehäuseabschnitt, der mit dem vorderen Gehäuseabschnitt lösbar verbunden ist. Der vordere Gehäuseabschnitt ist mit einem ersten Verriegelungselement verbunden oder bildet das erste Verriegelungselement, und der hintere Gehäuseabschnitt ist mit einem zweiten Verriegelungselement verbunden oder bildet das zweite Verriegelungselement. Diese wenigstens zwei Verriegelungselemente stehen im verbundenen Zustand der Gehäuseabschnitte in einem Verriegelungseingriff. In bevorzugter Ausführung werden die Gehäuseabschnitte durch den Verriegelungseingriff in Bezug auf die Vorschubrichtung, d.h. axial, aneinander festgelegt. Sie können durch den Verriegelungseingriff stattdessen oder nur zusätzlich auch verdrehgesichert bezüglich einer Drehbewegung um eine zur Vorschubrichtung parallele Längsachse der Gehäuseabschnitte aneinander festgelegt werden. Der Verriegelungseingriff kann die einzige Verbindung der Gehäuseabschnitte bilden. Mittels der Verriegelungselemente kann ein Formschluss lediglich zusätzlich zu einer weiteren, gegebenenfalls andersartigen Verbindung der Gehäuseabschnitte gebildet sein.

Das Verabreichungsgerät umfasst ferner eine Kolbenstange, die dazu dient, den Kolben in die Vorschubrichtung zu bewegen. Die Kolbenstange kann mit dem Kolben fest, d.h. ständig, verbunden sein, worunter auch eine einstückige Ausbildung von Kolben und Kolbenstange verstanden werden soll. In bevorzugter Ausführung sind der Kolben und die Kolbenstange jedoch als separate Bauteile ausgeführt, und es drückt zum Zwecke der Produktausschüttung die Kolbenstange mit einem vorderen Ende gegen eine Rückseite des Kolbens.

Von dem zweiten Gehäuse wird in und gegen die Vorschubrichtung bewegbar ein Antriebselement einer Antriebsvorrichtung oder einer Dosier- und Antriebsvorrichtung gelagert, das mit der Kolbenstange so gekoppelt ist, dass es nur bei seiner Bewegung in die Vorschubrichtung die Kolbenstange mitnimmt, nicht jedoch bei seiner Rückbewegung entgegen der Vorschubrichtung.

Schließlich umfasst das Verabreichungsgerät eine Verriegelungssperre für die Verriegelungselemente. Die Verriegelungssperre ist mit dem Antriebselement so gekoppelt, dass der Verriegelungseingriff der Verriegelungselemente nur gelöst werden kann, wenn das Antriebselement eine Freigabestellung einnimmt. Vorzugsweise blockiert die Verriegelungssperre die in Verriegelungseingriff befindlichen Verriegelungselemente in allen Stellungen, die das Antriebselement im Rahmen seiner Bewegung in und gegen die Vorschubrichtung einnehmen kann, allerdings mit Ausnahme der Freigabestellung. Besonders bevorzugt entspricht die Freigabestellung des Antriebselements einer vordersten Stellung, die das Antriebselement nach dem Zusammenbau des Geräts in die Vorschubrichtung einnehmen kann.

Indem die Verriegelungselemente mit dem Antriebselement erfindungsgemäß gekoppelt sind, nämlich mittels der Verriegelungssperre, kann sichergestellt werden, dass bei einem Verabreichungsgerät, das die Auswahl der zu verabreichenden Produktdosis erlaubt, diese Dosisauswahl aus einem Zustand heraus erfolgt, der auch tatsächlich einer Nulldosis entspricht. Ohne die erfindungsgemäße Maßnahme bestünde nämlich die Gefahr, dass bei einer versehentlichen ersten Betätigung des Antriebselements nach dem Zusammenbau Produkt ausgeschüttet wird, obgleich der Verwender noch gar keine Produktdosis ausgewählt hat. Hätte er eine Produktdosis ausgewählt, so bestünde die Gefahr, dass über die ausgewählte Produktdosis hinaus noch eine Zusatzmenge an Produkt ausgeschüttet wird.

Die erfindungsgemäße Kopplung des Antriebselements mit dem Verriegelungsmechanismus ist jedoch auch bei Verabreichungsgerät von Vorteil, die zwar nicht die Auswahl der Dosis, aber den Austausch eines Produktreservoirs beispielsweise in Form einer Austauschampulle erlauben. Bei solchen Geräten, bei denen die ausschüttbare Produktmenge fest vorgegeben ist, kann durch die erfindungsgemäße Ausbildung beispielsweise sichergestellt werden, dass bei dem Zusammenbau der Gehäuse ein Primevorgang durchgeführt wird, was auch für Geräte mit Dosisauswahl vorteilhaft sein kann.

Falls das Verabreichungsgerät eine Dosisanzeige, beispielsweise eine LCD Anzeige, aufweist, kann mittels der Kopplung des Antriebselements mit dem Verriegelungs-Mechanismus eine Nullung der Anzeige bewirkt werden. Obwohl eine solche Nullung auch mechanisch bewerkstelligt werden kann, wird die Verriegelungssperre hierfür bevorzugt zu einem Schalter in einem beispielsweise elektronischen Schaltkreis, der die Anzeige umfasst, weitergebildet.

Falls das Verabreichungsgerät eine infundierende Kanüle aufweist, hat diese Kanüle bevorzugter Weise einen Außendurchmesser, der dem einer Kanüle von 30 Gauge entspricht oder vorzugsweise kleiner ist. Eine Kanüle von 31 Gauge oder eine noch dünnere Kanüle sind besonders bevorzugte Beispiele. Auch Kanülen mit Außendurchmessern und/oder Innendurchmessern, die nicht der ISO 9626 Norm entsprechen, aber deren Außendurchmesser nicht größer als derjenige einer Kanüle von 30 Gauge sind, stellen bevorzugte Ausführungsbeispiele dar, insbesondere wenn die Wandstärken solcher Kanülen geringer als die nach der Norm spezifizierten sind. Obgleich die Kanülenabmessungen mit Bezug auf die ISO 9626 beschrieben sind, die für Stahlkanülen gilt, sollen die vorstehenden Ausführungen jedoch gleichermaßen auch für Kanülen aus anderen biokompatiblen Materialien gelten.

In bevorzugten Ausführungen gestattet das Gerät, wie bereits erwähnt, die Verabreichung mehrerer, jeweils einzeln auswählbarer Produktdosen. In dieser Ausbildung umfasst es vorzugsweise ein Dosiseinstellglied, das mit der Kolbenstange in solch einem Eingriff steht, dass es relativ zu dem vorderen und/oder relativ zu dem hinteren Gehäuseabschnitt in die Vorschubrichtung und relativ zu diesem Gehäuseabschnitt und der Kolbenstange gegen die Vorschubrichtung bewegbar ist, um durch einen Bewegung gegen die Vorschubrichtung die Produktdosis auszuwählen und bei einer Bewegung in die Vorschubrichtung die Kolbenstange mitzunehmen. Das Antriebselement wirkt in dieser Ausbildung vorzugsweise auf das Dosiseinstellglied. Durch den Verriegelungseingriff und dessen Blockierung und Freigabe mittels der Verriegelungssperre wird sichergestellt, dass mit der Herstellung des Verriegelungseingriffs auch das Dosiseinstellglied in eine definierte Stellung in Bezug auf den Kolben bewegt wird. Diese definierte Stellung ist vorzugsweise die Nullstellung des Dosiseinstellglieds, in der mittels des Antriebselements über das Dosiseinstellglied nicht auf den Kolben eingewirkt werden kann.

In der zuletzt geschilderten Ausbildung des Geräts als Gerät zur Verabreichung einer auswählbaren Produktdosis werden die Kolbenstange und das Dosiseinstellglied vorzugsweise von dem vorderen Gehäuseabschnitt, besonders bevorzugt in dem vorderen Gehäuseabschnitt, gelagert und bilden mit dem vorderen Gehäuseabschnitt ein Reservoirmodul. Dieses Reservoirmodul ist in bevorzugten Ausführungen als Wegwerfmodul konzipiert, d.h. das Reservoirmodul wird entsorgt, sobald das Reservoir entleert worden ist. Falls die Kolbenstange oder ein Dosiseinstellglied nicht von dem vorderen Gehäuseabschnitt gelagert wird, sondern beispielsweise von dem hinteren Gehäuseabschnitt, wird das Reservoirteil, welches den vorderen Gehäuseabschnitt und das Reservoir einschließlich Kolben umfasst, bereits alleine ebenfalls als Reservoirmodul im Sinne der Erfindung verstanden.

Der vordere Gehäuseabschnitt ist vorzugsweise zweiteilig und umfasst das Reservoirteil und einen Mechanikhalter. Falls eine Ampulle das Reservoir bildet, wird das Reservoirteil als Ampullenhalter bezeichnet. Der Mechanikhalter dient der Lagerung der Kolbenstange und vorzugsweise auch des Dosiseinstellglieds. Er wird mit dem Reservoirteil verschiebegesichert und vorzugsweise auch verdrehgesichert verbunden. Die Verbindung kann lösbar, beispielsweise als Schraubverbindung, ausgebildet oder unlösbar sein. Es kann auch der das Reservoir aufnehmende vordere Gehäuseabschnitt für eine mehrmalige Verwendung vorgesehen und nur das Reservoir austauschbar sein. Grundsätzlich gilt dies auch für den Mechanikhalter, falls ein solcher mit entsprechender Mechanik vorgesehen ist. Die Konzeption eines Reservoirmoduls als Wegwerfteil weist allerdings aufgrund der üblicherweise verwendeten rückzugsgesicherten Kolbenstangen den Vorteil der bequemen Handhabung auf.

Der Verriegelungseingriff der Sperrelemente hat bei dem zusammengebauten Gerät zwar zur Folge, dass der vordere Gehäuseabschnitt und der hintere Gehäuseabschnitt nur in der Freigabestellung des Antriebselements voneinander getrennt werden können. Der Hauptzweck ist jedoch darin zu sehen, dass der vordere Gehäuseabschnitt und der hintere Gehäuseabschnitt nur in der Freigabestellung des Antriebselements miteinander verbunden werden können, um nämlich einen definierten Zustand, bevorzugt den Nulldosis-Zustand, durch den Zusammenbau herzustellen oder nur ein Primen durchzuführen. Dementsprechend betrifft die Erfindung auch ein den vorderen Gehäuseabschnitt mit dem ersten Verriegelungselement umfassendes Reservoirmodul allein. Eine Ausführungsform betrifft ferner auch eine den hinteren Gehäuseabschnitt mit dem zweiten Verriegelungselement und das Antriebselement umfassende Antriebsvorrichtung, die zusätzlich auch eine Dosiervorrichtung oder zumindest einen Teil einer Dosiervorrichtung beinhalten kann und in diesem Falle zu einer Dosier- und Antriebsvorrichtung weitergebildet ist.

Die Verriegelungssperre ist vorzugsweise in oder an der Antriebsvorrichtung bzw. Dosier- und Antriebsvorrichtung ausgebildet, kann jedoch stattdessen auch in oder an dem Reservoirmodul vorgesehen sein.

Das erste Verriegelungselement und/oder das zweite Verriegelungselement kann ein elastischer Schnapper sein, der durch elastisches Nachgeben in und aus dem Verriegelungseingriff bewegt wird. Die Verriegelungsvorrichtung würde das elastische Nachgeben nur in der Freigabestellung des Antriebselements zulassen. In bevorzugter Ausbildung ist jedoch ein für die Herstellung und das Lösen des Verriegelungseingriffs bewegbar mit seinem zugeordneten Gehäuseabschnitt verbundenes Verriegelungselement in sich steif oder doch zumindest so steif ist, dass ein zur Lösung des Verriegelungseingriffs führendes elastisches Nachgeben des Verriegelungselements in sich nicht möglich ist. Vielmehr wird das bewegliche Verriegelungselement gegen die Kraft eines elastischen. Rückstelleinrichtung, vorzugsweise gegen die Kraft einer Druckfeder, in und aus dem Verriegelungseingriff bewegbar an dem zugeordneten Gehäuseabschnitt abgestützt: Die Bewegung eines elastischen und auch die Bewegung eines vorzugsweise nicht elastischen Veniegelungselements ist quer, vorzugsweise rechtwinklig, zu der Vorschubrichtung gerichtet. Besonders bevorzugt weisen die Verriegelungsbewegung und Entriegelungsbewegung radial zu einer zentralen Längsachse des Geräts.

Die wenigstens zwei, vorzugsweise genau zwei, in und aus dem Verriegelungseingriff bringbaren Verriegelungselemente werden vorzugsweise von einem männlichen Verriegelungselement und einem aufnehmenden, weiblichen Verriegelungselement in der Art einer Schloss/Riegel-Verbindung gebildet.

Die Verriegelungssperre umfasst vorzugsweise einen Sperrschieber, der das bewegbare Verriegelungselement - und im Falle von zwei bewegbaren Verriegelungselementen wenigstens eines der Verriegelungselemente oder gegebenenfalls beide Verriegelungselemente - blockiert. Die Blockierung ist derart, dass das blockierte Verriegelungselement eine Entriegelungsbewegung nicht ausführen kann, solange das Antriebselement die wenigstens eine, vorzugsweise genau eine Freigabestellung nicht einnimmt. Die Verriegelungssperre ist mit dem Antriebselement vorzugsweise steif verbunden, so dass sie die Bewegungen des Antriebselements 1:1 mitmacht. Das Antriebselement und die Verriegelungssperre können als ein einziges Bauteil, d.h. einstückig, ausgebildet sein, vorzugsweise ist oder umfasst die Verriegelungssperre jedoch ein von dem Antriebselement separates Teil, das mit dem Antriebselement so verbunden ist, dass es dessen Bewegung in Vorschubrichtung und vorzugsweise auch dessen Bewegung entgegen der Vorschubrichtung 1:1 mitmacht. Die Sperrbewegung und Entsperrbewegung der Verriegelungssperre und die Bewegung des wenigstens einen, vorzugsweise genau einen, von ihr gesperrten Verriegelungselements sind vorzugsweise quer, beispielsweise rechtwinklig, zueinander gerichtet.

Obgleich die Erfindung in erster Linie bei Verabreichungsgeräten Anwendung findet, bei denen die Bewegung des Antriebselements in Vorschubrichtung vom Verwender manuell und meist in einem Zuge durchgeführt wird, kann sie auch bei Infusionsgeräten mit Vorteil eingesetzt werden, um die gleiche Bewegung vergleichsweise langsam, kontinuierlich oder in kleinen Schritten motorisch auszuführen.

Die Dosier- und Antriebsvorrichtung kann manuell, halb automatisch oder voll automatisch arbeiten. Im ersten Fall wird sowohl die rotatorische Dosierbewegung als auch die translatorische Ausschüttbewegung manuell ausgeführt. Im zweiten Fall wird entweder die Dosierbewegung oder die Ausschüttbewegung manuell ausgeführt, und die andere Bewegung wird motorisch oder mittels einer anderen Art der Kraftbeaufschlagung, beispielsweise mittels Federkraft bewirkt, wenn der Verwender die entsprechende Bewegung durch einen Betätigungshandgriff ausgelöst hat. Im dritten Fall, der vollautomatischen Dosier- und Antriebsvorrichtung werden die Dosierbewegung und die Ausschüttbewegung motorisch oder mittels einer anderen Kraft, beispielsweise Federkraft, bewirkt. Es wird in diesem Fall manuell lediglich die Dosis ausgewählt, beispielsweise mittels einer oder mehreren Tasten, und es wird die Ausschüttbewegung ebenfalls durch einen eigenen, entsprechenden Betätigungshandgriff des Verwenders ausgelöst. In den meisten Ausführungen ist das erfindungsgemäße Verabreichungsgerät mit einer manuellen Dosier- und Antriebsvorrichtung ausgerüstet, die dann als Dosier- und Betätigungsvorrichtung bezeichnet wird. Soweit daher von einer Dosier- und Betätigungsvorrichtung die Rede ist, wird damit die manuelle Ausführung bezeichnet. Soweit von einer Dosier- und Antriebsvorrichtung die Rede ist, soll hierdurch keine Beschränkung in Bezug auf manuell, halb automatisch oder voll automatisch vorgenommen werden, sondern es soll jede der Ausführungen umfasst sein. Der Begriff "Dosier- und Betätigungsmodul" wird jedoch im Zusammenhang mit sämtlichen Ausführungen der Dosier- und Antriebsvorrichtung verwendet.

Die Dosier- und Antriebsvorrichtung kann ein Dosierelement, das die Dosierbewegung ausführt, und separat ein Antriebselement aufweisen, das die Ausschüttbewegung ausführt. Vorzugsweise werden die Dosierbewegung und Ausschüttbewegung jedoch von dem gleichen Körper der Dosier- und Antriebsvorrichtung ausgeführt, der im Folgenden daher auch als Dosier- und Antriebselement oder als Dosier- und Betätigungselement bezeichnet wird.

Bei dem Produkt handelt es sich vorzugsweise um ein Fluid, besonders bevorzugt eine Flüssigkeit, für eine medizinische, therapeutische, diagnostische, pharmazeutische oder kosmetische Anwendung. Das Produkt kann beispielsweise Insulin, ein Wachstumshormon oder auch dünn- bis dickflüssige, breiförmige Nahrung sein. Das Verabreichungsgerät wird vorzugsweise in solchen Verwendungen eingesetzt, in denen ein Verwender sich das Produkt selbst verabreicht, wie es beispielsweise in der Diabetestherapie üblich ist. Die Verwendung im stationären und ambulanten Bereich durch geschultes Personal soll jedoch nicht ausgeschlossen sein.

Die Produktverabreichung kann bei einem injektionsgerät mittels Injektionskanüle oder beispielsweise einer Düse für nadellose Injektionen erfolgen. Die Injektion oder Infusion kann insbesondere subkutan oder venös vorgenommen werden, oder auch intramuskulär. Im Falle der Verabreichung per Inhalation kann die ausgewählte Produktdosis aus dem Reservoir beispielsweise in eine Kammer des Inhalationsgeräts ausgeschüttet und mittels einer Zerstäubungseinrichtung für die Inhalation zerstäubt werden. Denkbar ist ferner eine orale Einnahme oder eine Verabreichung über die Speiseröhre, um nur einige Beispiele der Verabreichung zu nennen.

Besonders bevorzugt ist das Verabreichungsgerät semidisposable. In diesem Fall ist der vordere Gehäuseabschnitt Träger eines Reservoirmoduls, das nach Entleerung des Reservoirs entsorgt oder in einen Kreislauf zurückgeführt wird, und der hintere Gehäuseabschnitt ist Träger eines Dosier- und Betätigungsmoduls, das in Verbindung mit einem neuen Reservoirmodul wiederholt verwendbar ist. Da das Reservoirmodul als Verbrauchsmodul auch separat gehandelt wird, ist es auch separat Gegenstand der Erfindung.

Auch das Dosier- und Betätigungsmodul kann ein separater Gegenstand sein. Bei einem System aus einem Verabreichungsgerät und wenigstens einem Reservoirmodul kann das Reservoirmodul das Reservoirmodul des Geräts nach Verbrauch ersetzen.

Das Konzept des zweigeteilten Verabreichungsgeräts in einen für die nur einmalige Verwendung vorgesehenen Teil und einen für die wiederholte Verwendung vorgesehenen Teil (semidisposable) ist vorteilhaft insbesondere für Injektionspens, aber desweiteren auch für beispielsweise Inhalationsgeräte oder Geräte für die orale Einnahme eines Produkts oder eine künstliche Ernährung.

In den Unteransprüchen werden weitere bevorzugte Ausgestaltungen der Erfindung beschrieben, wobei Merkmale die nur in Bezug auf das Verabreichungsgerät oder nur in Bezug auf ein Reservoirmodul oder ein Dosier- und Betätigungsmodul beansprucht sind, auch bevorzugte Merkmale in Bezug auf den jeweils anderen Anspruchsgegenstand sind.

Ausführungsbeispiele der Erfindung werden nachfolgend anhand von Figuren beschrieben. An den Ausführungsbeispielen offenbar werdende Merkmale bilden je einzeln und in jeder Merkmalskombination die Gegenstände der Ansprüche vorteilhaft weiter. Auch Merkmale, die nur an dem einen Beispiel offenbart sind, bilden das jeweils andere Beispiel weiter oder zeigen eine Alternative auf, soweit nichts Gegenteiliges offenbart wird oder nur der Fall sein kann. Es zeigen:
- Figur 1: zwei Teile eines Reservoirmoduls nach einem ersten Ausführungsbeispiel,
- Figur 2: das aus den zwei Teilen der Figur 1 erhaltene Reservoirmodul,
- Figur 3: ein das Reservoirmodul der Figur 2 umfassendes Injektionsgerät nach dem ersten Ausführungsbeispiel in einem Längsschnitt,
- Figur 4: einen Teil des Injektionsgeräts der Figur 3,
- Figur 5: einen Mechanikhalter des Reservoirmoduls in einem Längsschnitt und zwei Ansichten,
- Figur 6: eine von dem Mechanikhalter gelagerte Blockiereinrichtung für eine Kolbenstange,
- Figur 7: eine Kolbenstange in einem Längsschnitt und einer Stirnansicht,
- Figur 8: eine Verriegelungssperre in einem Längsschnitt, einer Ansicht und einer Draufsicht,
- Figur 9: ein zweites Ausführungsbeispiel eines Injektionsgeräts,
- Figur 10: den Querschnitt A-A der Figur 9,
- Figur 11: den Querschnitt B-B der Figur 9,
- Figur 12: den Querschnitt C-C der Figur 9,
- Figur 13: den Querschnitt D-D der Figur 9,
- Figur 14: den Mechanikhalter des zweiten Ausführungsbeispiels in einer perspektivischen Darstellung,
- Figur 15: den Mechanikhalter der Figur 14 in einer Ansicht,
- Figur 16: den Querschnitt A-A der Figur 15,
- Figur 17: das Dosiseinstellglied des zweiten Ausführungsbeispiels in einer perspektivischen Darstellung,
- Figur 18: das Dosiseinstellglied der Figur 17 in einem Längsschnitt,
- Figur 19: das Dosiseinstellglied der Figur 17 in einer Ansicht,
- Figur 20: das Dosiseinstellglied der Figur 17 in einer Draufsicht,
- Figur 21: einen Teil des Injektionsgeräts nach Figur 3 und
- Figur 22: einen Teil des Injektionsgeräts nach Figur 9.

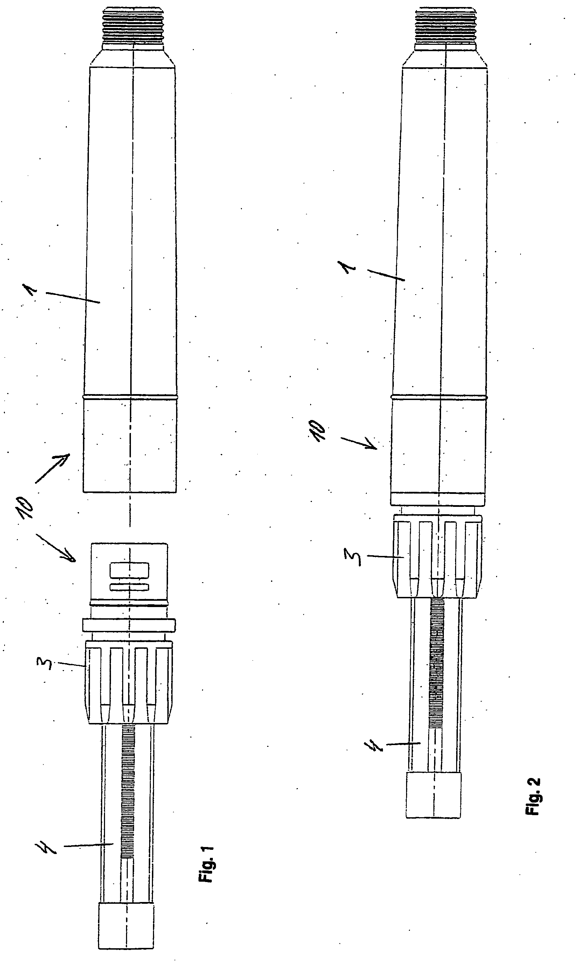

Figur 1 zeigt in einer Ansicht ein Reservoirteil 1 und einen Mechanikhalter 3, die miteinander verbunden werden, um das in Figur 2 dargestellte Reservoirmodul 10 zu bilden.

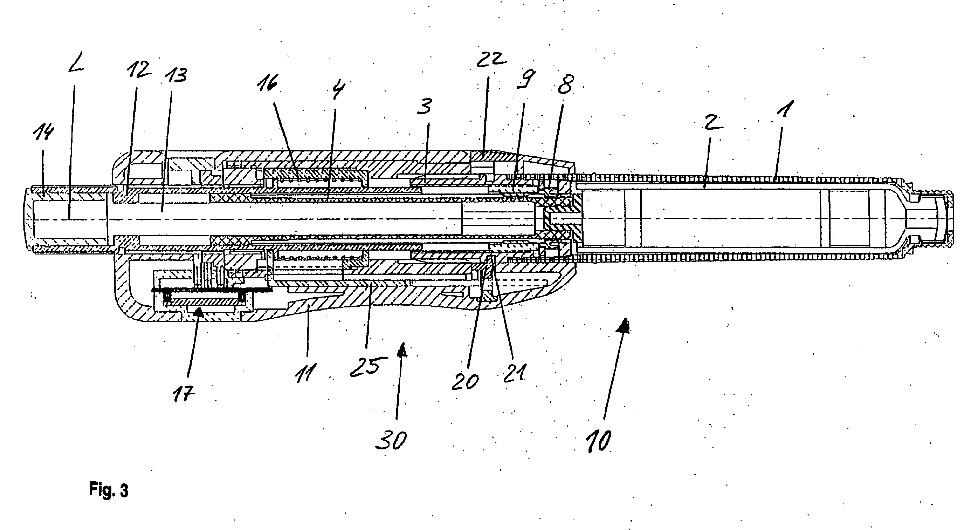

In den Figuren 1 und 2 ist ferner eine Kolbenstange 4 erkennbar, die an einem von dem Reservoirteil 1 abgewandten Ende des Mechanikhalters 3 in den Mechanikhalter 3 hineinragt und von dem Mechanikhalter 3 in eine in Längsachse L der Kolbenstange 4 weisende Vorschubrichtung auf ein von dem Mechanikhalter 3 abgewandtes, vorderes Ende des Reservoirteils 1 zu verschiebbar gelagert wird. Das Reservoirteil 1 ist im Wesentlichen ein im Querschnitt kreisförmiger Hohlzylinder, der an seinem vorderen Ende einen Verbindungsbereich für eine Verbindung mit einem Nadelhalter für eine Injektionsnadel aufweist. Das Reservoirteil 1 dient der Aufnahme eines Reservoirbehältnisses, das im Ausführungsbeispiel von einer Ampulle 2 gebildet wird, die in dem Längsschnitt der Figur 3 erkennbar ist. Ein Auslass an dem vorderen Ende der Ampulle 2 wird von einer Membran fluiddicht verschlossen. Bei der Befestigung des Nadelhalters an dem vorderen Ende des Reservoirteils 1 durchsticht ein rückwärtiger Teil der Injektionsnadel die Membran, so dass eine Fluidverbindung zwischen der Spitze der hohlen Injektionsnadel und dem Reservoir 2 hergestellt ist.

Figur 3 zeigt das Injektionsgerät in seiner Gesamtheit in einem Längsschnitt. In der Ampulle 2 ist ein Kolben in die Vorschubrichtung auf den am vorderen Ende der Ampulle 2 gebildeten Auslass zu verschiebbar aufgenommen. Durch die Verschiebung des Kolbens in Vorschubrichtung wird Produkt aus der Ampulle 2 verdrängt und durch den Auslass und die Injektionsnadel hindurch ausgeschüttet.

Den Vorschub des Kolbens bewirkt die Kolbenstange 4, die mit ihrem vorderen Ende gegen den Kolben drückt und dadurch bei ihrem eigenen Vorschub den Kolben in die Vorschubrichtung bewegt. Die Kolbenstange 4 wird von dem Mechanikhalter 3 so gehalten, dass sie nach Überwindung eines gewissen Widerstands in die Vorschubrichtung, nicht jedoch entgegen der Vorschubrichtung bewegt werden kann. Die Rückwärtsbewegung der Kolbenstange 4 entgegen der Vorschubrichtung wird von einer Blockiereinrichtung 8 verhindert. Die Blockiereinrichtung 8 wird von dem Mechanikhalter 3 axial fixiert, d.h. sie ist in dem Mechanikhalter 3 so gehalten, dass sie in und gegen die Vorschubrichtung nicht bewegbar ist. Allerdings wird sie von dem Mechanikhalter 3 um die Längsachse L drehbar gelagert. Die Blockiereinrichtung 8 erzeugt auch den Widerstand, der für die Vorwärtsbewegung überwunden werden muss.

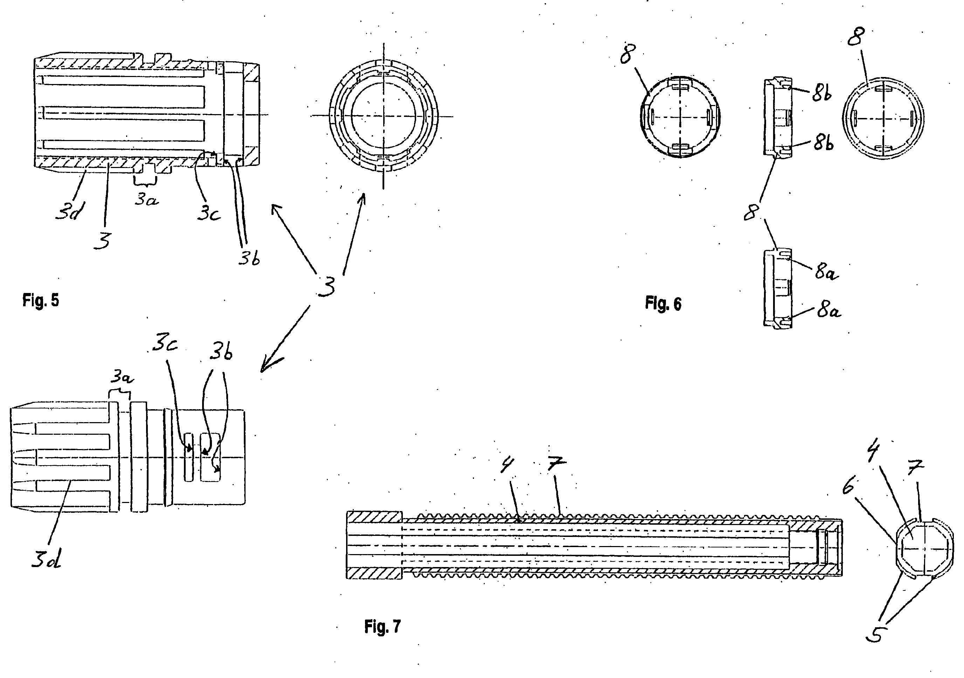

Die Blockiereinrichtung 8 ist in Figur 6 alleine dargestellt. Sie wird von einem einteiligen Ringelement gebildet, das an dem Mechanikhalter 3 zwischen zwei einander zugewandt beabstandeten Schultern 3b, die von einem Innenmantel des Mechanikhaltes 3 radial nach innen vorstehen, um die Längsachse L drehbar anliegt. Die Schultern 3b bilden eine Fixiereinrichtung für die axiale Fixierung der Blockiereinrichtung 8. Die Lagerung der Blockiereinrichtung 8 in dem Mechanikhalter 3 ist am besten aus der Darstellung des Mechanikhalters 3 in Figur 5 ersichtlich.

In dem Mechanikhalter 3 ist ferner ein Dosiseinstellglied 9 aufgenommen. Das Dosiseinstellglied 9 ist als Gewindemutter ausgebildet und steht mit einem Außengewinde der Kolbenstange 4 in einem Gewindeeingriff. Das Dosiseinstellglied 9 wird von dem Mechanikhalter 3 verdrehgesichert, aber in und gegen die Vorschubrichtung axial linear bewegbar geführt. Die Kolbenstange 4 und das Dosiseinstellglied 9 bilden einen Spindeltrieb, um die zu verabreichende Produktdosis auszuwählen.

Der Ampullenhalter 1 und der Mechanikhalter 3 sind verdreh- und verschiebegesichert miteinander verbunden und bilden zusammen das Reservoirmodul 10 des Injektionsgeräts, wobei dieses Reservoirmodul 10 die von dem Mechanikhalter 3 mittels der Blockiereinrichtung 8 gehaltene Kolbenstange 4 und das Dosiseinstellglied 9 umfasst. Der Ampullenhalter 1 und der Mechanikhalter 3 bilden zusammen einen vorderen Gehäuseabschnitt des Injektionsgeräts. Mit diesem vorderen Gehäuseabschnitt 1, 3 ist ein hinterer Gehäuseabschnitt 11 formschlüssig verbunden. Der hintere Gehäuseabschnitt 11 bildet den Träger eines Dosier- und Betätigungselements 12 und zusammen mit demselben sowie Teilen einer Verriegelungseinrichtung und weiteren Teilen ein Dosier- und Betätigungsmodul 30 des Injektionsgeräts.

Eine Dosier- und Betätigungsvorrichtung umfasst mit Ausnahme des Dosiseinstellglieds 9, der Kolbenstange 4 und der Blockiereinrichtung 8 die anderen Komponenten für die Auswahl der Produktdosis und die Betätigung des Injektionsgeräts. Insbesondere umfasst sie das Dosier- und Betätigungselement 12. Ferner umfasst die Dosier- und Betätigungsvorrichtung eine Zähl- und Anzeigeeinrichtung 17 zum Zählen und optischen Anzeigen der ausgewählten Produktdosis. Nicht zuletzt die Zähl- und Anzeigeeinrichtung 17 macht das Dosier- und Betätigungsmodul 30 zu einem hochwertigen und daher hochpreisigen Teil des Injektionsgeräts. Während das vergleichsweise preiswerte Reservoirmodul 10 als Einwegmodul konzipiert ist, ist das Dosier- und Betätigungsmodul 30 für die mehrmalige Verwendung mit immer wieder neuen Reservoirmodulen 10 bestimmt.

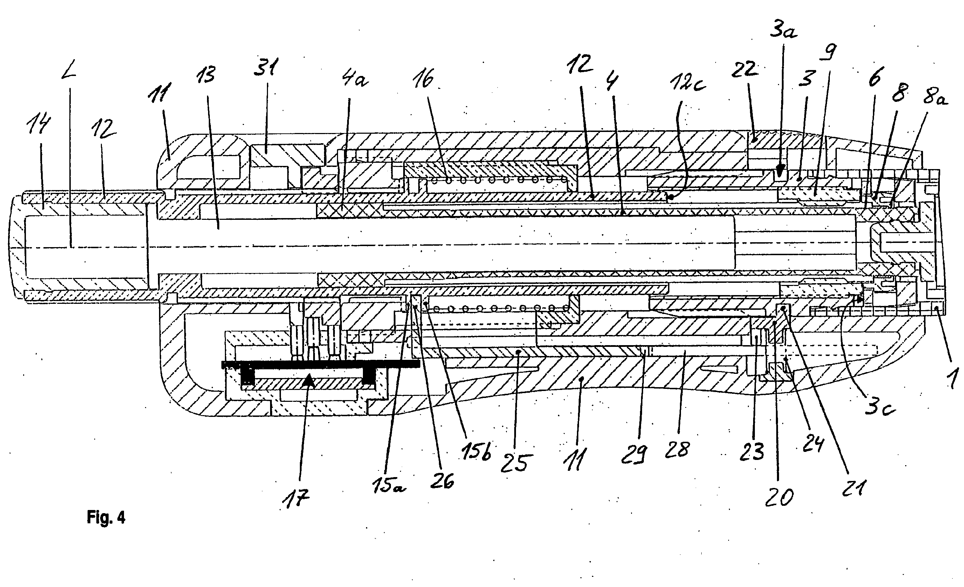

Für die Auswahl der Produktdosis, d.h. für die Dosierung, wird das Dosier- und Betätigungselement 12 um die Längsachse L drehbar und ferner in und gegen die Vorschubrichtung entlang der Längsachse L geradverschiebbar von dem hinteren Gehäuseabschnitt 11 gelagert. Das Dosier- und Betätigungselement 12 ist hohlzylindrisch und umgibt mit einem vorderen Abschnitt die Kolbenstange 4. Ein hinterer Abschnitt des Dosier- und Betätigungselements 12 ragt über ein hinteres Ende des Gehäuseabschnitts 11 hinaus. In das Dosier- und Betätigungselement 12 ist von hinten ein stangenförmiger Dosiermitnehmer 13 bis gegen eine radial nach innen vorragende Schulter des Dosier- und Betätigungselements 12 eingeschoben. Ferner ist an dem hinteren Ende ein Verschluss 14 bis gegen den Dosiermitnehmer 13 in das Dosier- und Betätigungselement 12 eingeschoben. Der Dosiermitnehmer 13 wird zwischen der radial vorragenden Schulter des Dosier- und Betätigungselements 12 und dem Verschluss 14 relativ zu dem Dosier- und Betätigungselement 12 axial fixiert. Der Dosiermitnehmer 13 ist mit dem Dosier- und Betätigungselement 12 außerdem verdrehgesichert verbunden. Der Dosiermitnehmer 13 ragt zum Zwecke der Dosierung von hinten in die hohle Kolbenstange 4 hinein. Die Kolbenstange 4 weist einen Verbindungabschnitt 4a auf (Fig. 4), der mit dem Dosiermitnehmer 13 in solch einem Eingriff ist, dass die Kolbenstande 4 und der Dosiermitnehmer 13 und mit diesem auch das Dosier- und Betätigungselement 12 relativ zueinander um die gemeinsame Längsachse L nicht verdrehbar, aber entlang der Längsachse L in und gegen die Vorschubrichtung relativ zueinander bewegbar sind. Hierfür ist der Verbindungsabschnitt 4a als Linearführung für den Dosiermitnehmer 13 ausgebildet.

Eine Rückstelleinrichtung 16 spannt das Dosier- und Betätigungselement 12 elastisch gegen die Vorschubrichtung in die in den Figuren 3 und 4 dargestellte Ausgangsstellung. In der Ausgangsstellung kann durch Drehung des Dosier- und Betätigungselements 12 um die Längsachse L die Dosierung vorgenommen werden. Anschließend kann aus der Ausgangsstellung heraus durch Axialverschiebung des Dosier- und Betätigungselements 12 die Ausschüttung der ausgewählten Produktdosis bewirkt werden. Die Rückstelleinrichtung 16 wird von einer als Druckfeder wirkenden Spiralfeder gebildet, die in einem Ringspalt um das Dosier- und Betätigungselement 12 aufgenommen ist und zwischen einer radial nach innen ragenden Schulter des Gehäuseabschnitts 11 und einer gegenüberliegend zugewandt radial nach außen ragenden Schulter des Dosier- und Betätigungselements 12 axial abgestützt ist.

Die Blockiereinrichtung 8 erfüllt eine Doppelfunktion. Zum einen stellt sie mit ihren Sperrelementen 8a sicher, dass die Kolbenstange 4 relativ zu dem Mechanikhalter 3 und damit insbesondere relativ zu dem in der Ampulle 2 aufgenommenen Kolben nicht entgegen der Vorschubrichtung zurückbewegt werden kann. In ihrer Doppelfunktion verhindert die Blockiereinrichtung 8 ferner als Bremse eine Vorwärtsbewegung der Kolbenstange 4 bei dem Dosiervorgang, bei dem das Dosiseinstellglied 9 entgegen der Vorschubrichtung axial auf das Dosier- und Betätigungselement 12 zu bewegt wird.

In der in den Figuren 3 und 4 dargestellten Ausgangsstellung vor einer Dosierung liegt das Dosiseinstellglied 9 in Vorschubrichtung auf Anschlag gegen einen von dem Mechanikhalter 3 gebildeten Ausschüttanschlag 3c (Figur 5). Die Kolbenstange 4 steht in einem permanenten Berührungskontakt mit dem Kolben. Zum Zwecke der Dosierung wird das Dosiseinstellglied 9 durch den Gewindeeingriff mit der Kolbenstange 4 und die Linearführung durch den Mechanikhalter 3 von dem Ausschüttanschlag 3c weg auf das Dosier- und Betätigungselement 12 zu bewegt. Hierdurch wird ein lichter Abstand zwischen einer rückwärtigen Anschlagfläche des Dosiseinstellglieds 9 und einer vorderen Anschlagfläche des Dosier- und Betätigungselements 12 verringert, andererseits jedoch der lichte Abstand zwischen einer vorderen Anschlagfläche des Dosiseinstellglieds 9 und dem Ausschüttanschlag 3c vergrößert. Der letztgenannte Abstand zwischen dem Ausschüttanschlag 3c und dem Dosiseinstellglied 9 ist die Weglänge, um die im Zuge der Ausschüttbewegung des Dosier- und Betätigungselements 12 das Dosiseinstellglied 9 und wegen des Gewindeeingriffs auch die Kolbenstange 4 in die Vorschubrichtung bewegt werden. Der Ausschüttanschlag 3c bildet einen vorderen Translationsanschlag. Bei der Ausschüttbewegung drückt die Kolbenstange 4 mit ihrem vorderen Ende, das von einem mit der Kolbenstange 4 in und gegen die Vorschubrichtung nicht bewegbar verbundenen Stempelkörper gebildet wird, gegen den Kolben und drückt den Kolben in die Vorschubrichtung auf den Auslass der Ampulle 2 zu vor. Die Längsachse L bildet die Rotations- und Translationsachse der Bewegungen, die zum Zwecke der Dosierung und Produktausschüttung ausgeführt werden.

Der Abstand, den das Dosiseinstellglied 9 und das Dosier- und Betätigungselement 12 vor dem Dosiervorgang zwischen sich aufweisen, wenn das Dosiseinstellglied 9 auf Anschlag gegen den Ausschüttanschlag 3c liegt, entspricht der maximalen Produktdosis, die ausgewählt und im Zuge einer Ausschüttung ausgeschüttet werden kann. Die Hubbewegung des Dosier- und Betätigungselements 12 ist bei jeder Ausschüttung gleich lang. Es wird durch die Dosierung lediglich der Abstand des Dosiseinstellglieds 9 von dem Ausschüttanschlag 3c und damit die im Zuge der Ausschüttung von dem Dosier- und Betätigungselement 12 und dem Dosiseinstellglied 9 gemeinsam zurücklegbare Weglänge eingestellt.

Die Bremsfunktion der Blockiereinrichtung 8 und der hierfür zwischen der Kolbenstange 4 und der Blockiereinrichtung 8 bestehende Bremseingriff werden aus der Zusammenschau der Figuren 6 und 7 deutlich. Zum einen weist die Blockiereinrichtung 8 für den Bremseingriff zwei Bremselemente 8b auf; die, wie bereits die Sperrelemente 8a, je von einem elastisch nachgebenden Schnapper gebildet werden. Die Blockiereinrichtung 8 wird im Ausführungsbeispiel von einem einzigen Ringelement gebildet, von dem an einer Stirnseite vier elastische Schnapper axial abragen. Die Schnapper sind gleichmäßig über den Umfang des Ringelements verteilt angeordnet. Zwei einander gegenüberliegende Schnapper bilden die Sperrelemente 8a und die zwei weiteren, einander ebenfalls gegenüberliegend angeordneten Schnapper bilden die Bremselemente 8b.

Die Kolbenstange 4 weist dementsprechend zwei am Außenmantel an gegenüberliegenden Seiten ausgebildete und in Längsrichtung der Kolbenstange 4 sich erstreckende Rückzugsperreinrichtungen 6 und zwei an ebenfalls einander gegenüberliegenden Seiten in Längsrichtung der Kolbenstange 4 sich erstreckende Vorschubbremseinrichtungen 7 auf. Das Gewinde der Kolbenstange 4 für den Gewindeeingriff mit dem Dosiseinstellglied 9 wird von vier verbleibenden Gewindeabschnitten 5 gebildet, die sich über nahezu die gesamte Länge der Kolbenstange 4 erstrecken. Die Rückzugsperreinrichtungen 6 und die Vorschubbremseinrichtungen 7 werden je von einer Zahnreihe gebildet. Während die Zähne der Rückzugsperreinrichtungen 6 jedoch als in Vorschubrichtung gepfeilte Sägezähne mit nach hinten weisenden, quer zur Vorschubrichtung sich erstreckenden Sperrflächen gebildet sind, weisen die beiden Zahnreihen, die die Vorschubbremseinrichtungen 7 bilden, nach vorne weisende Sperrflächen mit einer vergleichbaren Sperrwirkung nicht auf. Die Zähne der Vorschubbremseinrichtungen 7 weisen je ein im Vergleich zu den Rückzugssperreinrichtungen 6 weicheres Zahnprofil auf. Der Bremseingriff der Blockiereinrichtung 8 mit den Vorschubbremseinrichtungen 7 der Kolbenstange 4 soll eine Vorschubbewegung der Kolbenstange 4 nämlich nicht verhindern, sondern nur erschweren, um sicherzustellen, dass die Kolbenstange 4 nicht bei der Dosierung in Vorschubrichtung bewegt wird. Die Zähne der Vorschubbremseinrichtungen 7 sind an ihren Vorderseiten und die Bremselemente 8b sind an ihren Rückseiten, die die Vorderseiten der Zähne der Vorschubbremseinrichtung 7 berühren, so geformt, dass zur Überwindung des Bremseingriffs eine Schwellkraft überwunden werden muss, die bei der Dosierung nicht erreicht wird. Diese Schwellkraft ist größer als die Kraft, die erforderlich ist, um die Zähne der Rückzugssperreinrichtungen 6 über die Sperrelemente 8a in Vorschubrichtung zu bewegen. Vorzugsweise ist die Schwellkraft wenigstens doppelt so groß wie die anfängliche Reibkraft zwischen den Rückzugssperreinrichtungen 6 und den Sperrelementen 8a. Die Reibkraft zwischen letzteren vergrößert sich auch erst im Verlaufe der Vorschubbewegung allmählich zwischen zwei aufeinanderfolgenden Sperreingriffen. Die Schwellkraft des Bremseingriffs muss hingegen in jedem Sperreingriff unmittelbar am Beginn der Vorschubbewegung von einem Sperreingriff in den nächst folgenden aufgebracht werden. Allerdings soll die Schwellkraft nicht so groß sein, dass sie vom Verwender bei der Ausschüttung als störend empfunden wird.

Eine unerwünschte Vorschubbewegung der Kolbenstange als Reaktion auf die Bewegung des Dosiseinstellglieds 9 bei der Dosisauswahl kann grundsätzlich auch allein durch den Sperreingriff der Blockiereinrichtung 8 bewirkt werden. Allerdings wird solch eine Bewegung wegen des Bremseingriffs sicherer verhindert als durch den Sperreingriff allein.

Die Verbindung zwischen dem Reservoirmodul 10 und dem Dosier- und Betätigungsmodul 30 ist formschlüssig. Zum einen besteht zwischen dem Mechanikhalter 3 und dem Gehäuseabschnitt 11 ein Verriegelungseingriff, der eine Relativbewegung in axialer Richtung verhindert. Über den Verriegelungseingriff hinaus sind der vordere Gehäuseabschnitt 1, 3 und der hintere Gehäuseabschnitt 11 unmittelbar aneinander axial linear geführt, um eine Relativdrehung bei dem Verbinden und im verbundenem Zustand zu verhindern. In Figur 5 sind die Axialführungen 3d des Mechanikhalters 3, die mit einem oder mehreren entsprechenden Eingriffselementen des hinteren Gehäuseabschnitts 11 die Linearführung bilden, gut zu erkennen. Die Axialführungen 3d werden von Führungsflächen an Führungsrippen gebildet; sie könnten auch von Führungsflächen in axial sich erstreckenden Vertiefungen gebildet werden. Auf diese Weise werden axiale Führungskanäle erhalten. Die Führungsrippen sind axial verjüngt, so dass in die Führungskanäle führende Einführtrichter für das eine oder die mehreren Eingriffselemente des hinteren Gehäuseabschnitts 11 gebildet werden. Zur noch besseren Zentrierung der Gehäuseabschnitte 1, 3 und 11 zu Beginn des Verbindens sind die Führungsrippen auch noch in radialer Richtung verjüngt. Das eine oder die mehreren Eingriffselemente des hinteren Gehäuseabschnitts 11 ist oder sind vorzugsweise wie die Axialabschnitte 3d an der Mantelgegenfläche, d.h. der Innenmantelfläche, des hinteren Gehäuseabschnitts 11 gebildet.

Der Verriegelungseingriff besteht zwischen einem weiblichen, ersten Verriegelungselement 3a des Mechanikhalters 3 (Figur 5) und einem Verriegelungsring 20, der mit dem hinteren Gehäuseabschnitt 11 radial bewegbar, aber axial nicht bewegbar verbunden ist. Der Verriegelungsring 20 bildet ein unmittelbar in das erste Verriegelungselement 3a radial eingreifendes männliches, zweites Verriegelungselement 21. Zwischen dem ersten Verriegelungselement 3a und dem zweiten Verriegelungselement 21 besteht eine Schloss/Riegel-Verbindung, die axiale Relativbewegungen zwischen dem Reservoirmodul 10 und dem Dosier- und Betätigungsmodul 30 verhindert.

Die Figuren 3 und 4 zeigen das Verriegelungselement 21 im Verriegelungseingriff mit dem Verriegelungselement 3a. Das Verriegelungselement 3a wird von einem Ringsteg und einer Nut gebildet, die an dem Außenmantel des Mechanikhalters 3 umlaufen. Der Ringsteg bildet eine hintere Seitenwand der Nut. Das zweite Verriegelungselement 21 wird von einem Nocken gebildet, der von dem Innenmantel des Verriegelungsrings 20 radial einwärts vorragt und im Verriegelungseingriff von einer Rückstelleinrichtung 24 radial einwärts über eine Innenmantelfläche des hinteren Gehäuseabschnitts 11 vorstehend in das aufnehmende Verriegelungselement 3a hineingedrückt wird. Der Verriegelungsring 20 ist in seiner Gesamtheit in Radialrichtung mittels der Rückstelleinrichtung 24 an einer von dem Gehäuseabschnitt 11 gebildeten Innenmantelfläche abgestützt, so dass die Rückstelleinrichtung 24 etwa in radialer Verlängerung des Verriegelungselements 21 gegen den Außenmantel des Verriegelungsrings 20 drückt. Der Verriegelungsring 20 umgibt den Mechanikhalter 3 und ist in seiner Gesamtheit gegen die rückstellende Kraft der Rückstelleinrichtung 24 radial hin und her bewegbar, so dass das zweite Verriegelungselement 21 in und aus dem Verriegelungseingriff mit dem ersten Verriegelungselement 3a bringbar ist. Der hintere Gehäuseabschnitt 11 bildet eine enge Gleitführung für die Radialbewegung des Verriegelungsrings 20. An seiner dem Verriegelungselement 21 radial gegenüberliegenden Seite bildet der Verriegelungsring 20 einen Entriegelungsknopf 22 für den Verwender. Zur radialen Führung der als Druckfeder ausgebildeten Rückstelleinrichtung 24 ragt von der dem Verriegelungselement 21 abgewandten Außenmantelfläche des Verriegelungsrings 20 ein Führungsnocken radial ab.

In Umfangsrichtung zu beiden Seiten dieses Führungsnockens und axial hinter dem Führungsnocken ragen von der Außenmantelfläche des Verriegelungsrings 20 ferner zwei Sperrnocken 23 ab, die in Radialrichtung nach außen gegen eine Verriegelungssperre 25 drücken. Aufgrund der Anlage der Sperrnocken 23 gegen die Verriegelungssperre 25 wird eine Radialbewegung des Verriegelungselements 21 verhindert, die zu einem Lösen des Verriegelungseingriffs führen könnte. Der zwischen den Verriegelungselementen 3a und 21 bestehende Verriegelungseingriff wird somit durch die Verriegelungssperre 25 gesichert. Diese Sicherung besteht in jeder Verschiebeposition des Dosier- und Betätigungselements 12 mit Ausnahme einer Freigabestellung, die das Dosier- und Betätigungselement 12 am Ende seiner Ausschüttbewegung einnimmt. Die Freigabestellung fällt daher mit der vordersten Verschiebeposition zusammen, die das Dosier- und Betätigungselement 12 dann einnimmt, wenn es im Zuge seiner Ausschüttbewegung an das Dosiseinstellglied 9 und das Dosiseinstellglied 9 seinerseits gegen den Ausschüttanschlag 3c des Mechanikhalters 3 anstößt. Solange das Dosier- und Betätigungsmodul 30 mit dem Reservoirmodul noch nicht verbunden ist, wird ein mechanischer Anschlag für das Dosier- und Betätigungselement 12 von einem Anschlagelement 31 der Dosier- und Betätigungsvorrichtung gebildet. Im Ausführungsbeispiel bildet ein Resethalterring, der einem Reset der Anzeige 17 dient, das Anschlagelement 31. Der Anschlag des Dosier- und Betätigungselement 12 gegen dieses Anschlagelement 31 definiert die Freigabestellung des Dosier- und Betätigungselements 12 in diesem Falle, wobei die durch das Anschlagelement 31 definierte Freigabestellung derjenigen entspricht, die durch das Anstoßen des Dosiseinstellglieds 9 an dem Ausschüttanschlag 3c definiert wird.

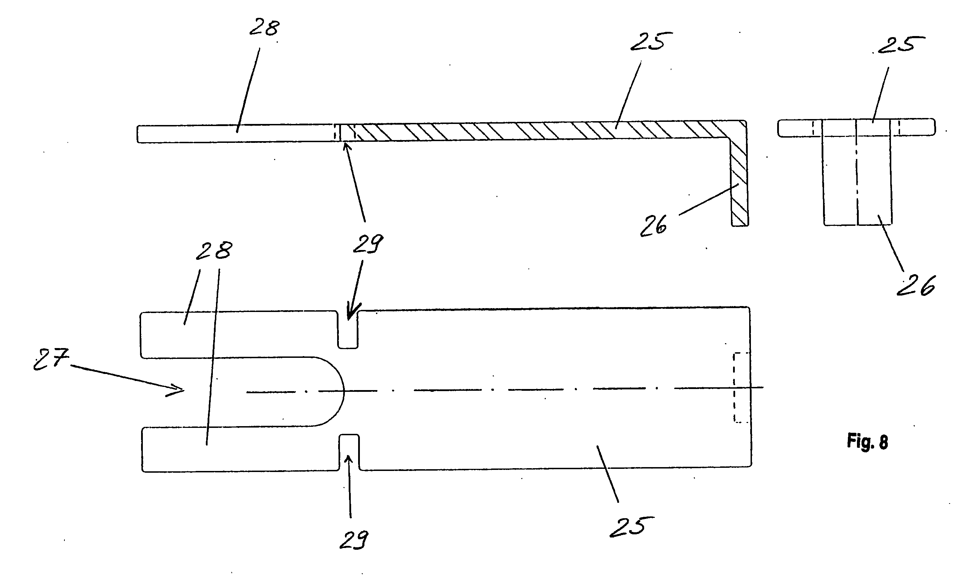

Figur 8 zeigt die Verriegelungssperre 25. Sie wird im Ausführungsbeispiel einstückig von einem Sperrschieber gebildet. Die Verriegelungssperre 25 weist einen plattenförmigen Hauptkörper auf, der sich im montierten Zustand, wie beispielsweise in Figur 4 dargestellt, axial erstreckt. An einem Ende ragt von dem Hauptkörper ein Steg 26 rechtwinklig ab. Im montierten Zustand erstreckt sich der Steg 26 radial bis gegen das Dosier- und Betätigungselement 12. Der Steg 26 dient der Befestigung der Verriegelungssperre 25 an dem Dosier- und Betätigungselement 12, das hierfür zwei axial beabstandet an einer Außenmantelfläche gebildete Ringstege aufweist, die Mitnehmer 15a und 15b bilden. Der vordere Mitnehmer 15b bildet gleichzeitig die Stützschulter für die Rückstelleinrichtung 16. In den zwischen den Mitnehmern 15a und 15b gebildeten Ringraum ragt die Verriegelungssperre 25 mit ihrem Steg 26 ein und wird von den beiden Mitnehmern 15a und 15b axial beidseits eng eingefasst.

An einem von dem Steg 26 abgewandten vorderen Ende ist der Hauptkörper der Verriegelungssperre 25 mit einer Axialausnehmung 27 versehen, die zu dem vorderen Ende der Verriegelungssperre 25 hin offen ist. Auf diese Weise werden beidseits der Ausnehmung 27 axial sich erstreckende Sperrzungen 28 gebildet. Die beiden Sperrnocken 23 des Verriegelungsrings 20 sind so angeordnet, dass je einer dieser Sperrnocken 23 gegen eine der Sperrzungen 28 drückt, solange das Dosier- und Betätigungselement 12 nicht die Freigabestellung einnimmt. Durch die axiale Ausnehmung 27 hindurch erstreckt sich bei der Axialbewegung der Verriegelungssperre 25 die Rückstelleinrichtung 24 für das Verriegelungselement 21.

In dem Hauptkörper der Verriegelungssperre 25 sind ferner Einrückausnehmungen 29 gebildet, die die Freigabestellung des Dosier- und Betätigungselements 12 definieren. Pro Sperrnocken 23 ist je eine Einrückausnehmung 29 vorgesehen. Die Position der Einrückausnehmungen 29 ist so gewählt, dass sie mit den Sperrnocken 23 erst dann in Überdeckung gelangen und dadurch ein Einfahren der Sperrnocken 23 gestatten, wenn das Dosier- und Betätigungselement 12 bis in seine Freigabestellung vorbewegt worden ist.

Es ist klar, dass bei der im Ausführungsbeispiel speziell gewählten Anordnung auch ein einziger Sperrnocken 23 vorgesehen sein könnte und die Verriegelungssperre 25 dementsprechend auch nur eine einzige Einrückausnehmung 29 und gegebenenfalls auch nur eine Sperrzunge 28 aufweisen könnte. Grundsätzlich könnte die Verriegelungssperre ferner einstückig mit dem Dosier- und Betätigungselement 12 gefertigt sein. Die Ausbildung als separates Teil bietet jedoch Vorteile hinsichtlich der Fertigung, der Montage und dem Zusammenwirken des Dosier- und Betätigungselements 12 mit der Kolbenstange 4. In Bezug auf die Einbaulage der Verriegelungssperre 25 ist noch darauf hinzuweisen, dass die Verriegelungssperre 25 an ihrer von dem Verriegelungselement 21 abgewandten Außenseite an einer Innenmantelfläche des Gehäuses 11 abgestützt ist. Auf diese Weise wird die Stabilität der Sicherung für den Verriegelungseingriff erhöht. Vorzugsweise bildet das Gehäuse 11 eine Axialführung für die Verriegelungssperre 25.

Im Folgenden wird die Funktionsweise des Injektionsgeräts beschrieben, wobei angenommen sei, dass ein neues Reservoirmodul 10 und ein bereits wenigstens einmal verwendetes Dosier- und Betätigungsmodul 30 zusammengebaut und anschließend eine erste Produktausschüttung vorgenommen werden.

Das Dosier- und Betätigungsmodul 30 und das neue Reservoirmodul 10 werden axial zueinander ausgerichtet, so dass ihre beiden Längsachsen miteinander fluchten. Anschließend wird das Reservoirmodul 10 mit seinem rückwärtigen Ende in das nach vorne offene Gehäuse 11 des Dosier- und Betätigungsmoduls 30 eingeführt.

Hierbei zentrieren sich der Gehäuseabschnitt 1, 3 und der Gehäuseabschnitt 11 an den verjüngten Enden der Führungsrippen 3d des Mechanikhalters 3. Während des Aufschiebens werden die beiden Gehäuseabschnitte in einer durch die Linearführung vorgegebenen Drehwinkelposition axial aneinander lineargeführt, bis die Gehäuseabschnitte 1, 3 und 11 eine Verbindungsendposition einnehmen, in der der Verriegelungseingriff der Verriegelungselemente 3a und 21 hergestellt werden kann oder von selbst sich einstellt.

Das Dosier- und Betätigungselement 12 ist in vorgegebenen Drehwinkelpositionen relativ zu dem hinteren Gehäuseabschnitt 11 gerastet. Die Linearführung der Gehäuseabschnitte 1, 3 und 11 und die Drehwinkelrastposition des Dosier- und Betätigungselements 12 sind so aufeinander abgestimmt, dass der verdrehgesicherte Eingriff des Dosier- und Betätigungselements 12 mit der Kolbenstange 4 in jeder Rastposition des Dosier- und Betätigungselements 12 und jeder Drehwinkelposition, in der die Gehäuseabschnitte 1, 3 und 11 aneinander lineargeführt sind, hergestellt wird.

Falls das Dosier- und Betätigungselement 12 sich in einer Axialposition relativ zu dem Gehäuseabschnitt 11 befindet, die hinter der Freigabestellung liegt, wird das Verriegelungselement 21 von der Verriegelungssperre 25 in seiner radial innersten Stellung gehalten. In dieser Stellung des Verriegelungselements 21 können das Dosier- und Betätigungsmodul 30 und das Reservoirmodul 10 nicht bis in die Verbindungsendstellung aufeinandergeschoben und deshalb auch nicht miteinander verbunden werden, da der am Außenmantel des Mechanikhalters 3 gebildete Ringsteg, der das erste Verriegelungselement 3a mitbildet, vorher gegen das zweite Verriegelungselement 21 auf Anschlag zu liegen kommt.

Der Ringsteg kann zu einem in tangentialer Richtung kurzen Radialvorsprung reduziert werden, wenn dafür gesorgt ist, dass die Gehäuseabschnitte 1, 3 und 11 nur in der Drehwinkellage zusammengebaut werden können, in der solch ein Vorsprung und das zweite Verriegelungselement 21 in einer axialen Flucht zu liegen kommen. Der Ringsteg oder Radialvorsprung könnte das erste Verriegelungselement 3a auch alleine bilden, da es die wesentliche Funktion des ersten Verriegelungselements 3a ist, die Herstellung der Verbindung zwischen dem Reservoirmodul 10 und dem Dosier- und Betätigungsmodul 30 nur zuzulassen, wenn das Dosier- und Betätigungselement 12 seine Freigabestellung einnimmt. Ist diese Bedingung erfüllt, so würde das Dosier- und Betätigungselement 12 bei der Herstellung der Verbindung zwischen dem Reservoirmodul 10 und dem Dosier- und Betätigungsmodul 30 sicherstellen, dass das Dosiseinstellglied 9 sich in seiner Dosiernullstellung befindet, in der es an dem Ausschüttanschlag 3c des Mechanikhalters 3 anstößt.

Um den die vorstehend erläuterte Bedingung erfüllenden Zustand herzustellen, drückt der Verwender das Dosier- und Betätigungselement 12 axial relativ zu dem hinteren Gehäuseabschnitt 11 bis in die Freigabestellung vor. In dieser Relativstellung zwischen Gehäuseabschnitt 11 und Dosier- und Betätigungselement 12 können die Sperrnocken 23 in die Einrückaufnahmen 29 der Verriegelungssperre 25 bewegt werden. Der Verwender drückt daher nicht nur das Dosier- und Betätigungselement 12 bis wenigstens in die Freigabestellung vor, sondern gleichzeitig auch mittels des Entriegelungsknopfs 22 das erste Verriegelungselement 20 aus dem Verriegelungseingriff. Das Reservoirmodul 10 kann nun axial über den Ringsteg des ersten Verriegelungselements 3a bewegt und weiter in den hinteren Gehäuseabschnitt 11 eingeführt werden. Der Verwender kann den Entriegelungsknopf 22 loslassen. Sobald das erste Verriegelungselement 21 mit dem zweiten Verriegelungselement 3a in Überdeckung gelangt, schnappt es aufgrund der Kraft der Rückstelleinrichtung 24 in das aufnehmende Verriegelungselement 3a ein, so dass der Verriegelungseingriff hergestellt ist. Das Reservoirmodul 10 und das Dosier- und Betätigungsmodul 30 sind nun in Bezug auf die Stellung des Dosiseinstellglieds 9 und der Kolbenstange 4 in definierter Weise miteinander verbunden. Falls das Dosiseinstellglied 9 vor Herstellung des Verriegelungseingriffs noch einen lichten Abstand von dem Ausschüttanschlag 3c aufgewiesen hat, ist dieser Abstand aufgrund der zum Herstellen der Verbindung erforderlichen Einwirkung des Dosier- und Betätigungselements 12 beseitigt. Eine damit einhergehende Produktausschüttung kann hingenommen werden und kann zum Zwecke des Primens der Injektionsnadel sogar erwünscht sein. Die Zähl- und Anzeigeeinrichtung 17 wird vorzugsweise dabei genullt.

In dem derart herbeigeführten, definierten Ausgangszustand kann der Verwender die Dosierung vornehmen. Die Dosierung erfolgt durch Drehung des Dosier- und Betätigungselements 12 um die Längsachse L und relativ zu dem Gehäuseabschnitt 11. Da der Dosiermitnehmer 13 mit dem Dosier- und Betätigungselement 12 verdrehgesichert verbunden ist und seinerseits verdrehgesichert in die Kolbenstange 4 eingreift, nimmt das Dosier- und Betätigungselement 12 bei der rotatorischen Dosierbewegung die Kolbenstange 4 mit. Aufgrund des Gewindeeingriffs zwischen der Kolbenstange 4 und dem Dosiseinstellglied 9 und der Linearführung des Dosiseinstellglieds 9 durch den Mechanikhalter 3 führt das Dosiseinstellglied 9 eine von der Gewindesteigung des gegenseitigen Gewindeeingriffs vorgegebene, axiale translatorische Dosierbewegung in Richtung auf das Dosier- und Betätigungselement 12 aus. Das Dosier- und Betätigungselement 12 bildet einen hinteren Translationsanschlag 12c, der die translatorische Dosierbewegung des Dosiseinstellglieds 9 begrenzt und dadurch den maximal einstellbaren Ausschütthub definiert.

Die Zähl- und Anzeigeeinrichtung 17 zählt die der Drehwinkelstellung des Dosier- und Betätigungselements 12 entsprechenden Dosiseinheiten und zeigt sie optisch an.

Nach Auswahl der gewünschten Produktdosis ist der Dosiervorgang beendet. Die Ausschüttung der gewählten Produktdosis wird mittels der in Vorschubrichtung des Kolbens weisenden Ausschüttbewegung des Dosier- und Betätigungselements 12 bewirkt. Im Zuge seiner Ausschüttbewegung stößt das Dosier- und Betätigungselement 12 gegen das Dosiseinstellglied 9 und nimmt es mit. Indem das Dosiseinstellglied 9 im Zuge der Ausschüttbewegung gegen den Ausschüttanschlag 3c des Mechanikhalters 3 stößt, werden die Ausschüttbewegungen des Dosier- und Betätigungselements 12 und die Produktausschüttung beendet. Nach dem Loslassen des Dosier- und Betätigungselements 12 vorzugsweise wird dieses von dem Rückstelleinrichtung 16 entgegen der Vorschubrichtung wieder in eine neue Ausgangsstellung für eine erneute Dosierung und Produktausschüttung bewegt. Die Zähl- und Anzeigeeinrichtung 17 ist mit dem Dosier- und Betätigungselement 12 vorzugsweise so gekoppelt, dass sie mittlerweile wieder auf Null zurückgestellt worden ist. Gegebenenfalls verfügt sie über Mittel zum Zählen und Anzeigen der bereits insgesamt ausgeschütteten Produktmenge und somit der in der Ampulle 2 verbliebenen Produktrestmenge.

Um das Reservoirmodul 10 von dem Dosier- und Betätigungsmodul 30 zu lösen, wird das Dosier- und Betätigungselement 12 bis in die Freigabestellung, d.h. bis auf Anschlag gegen das Dosiseinstellglied 9, vorbewegt. In dieser Stellung kann der Verwender durch Druck auf den Entriegelungsknopf 22 den Verriegelungseingriff wieder lösen und das Reservoirmodul 10 von dem Dosier- und Betätigungsmodul 30 trennen.

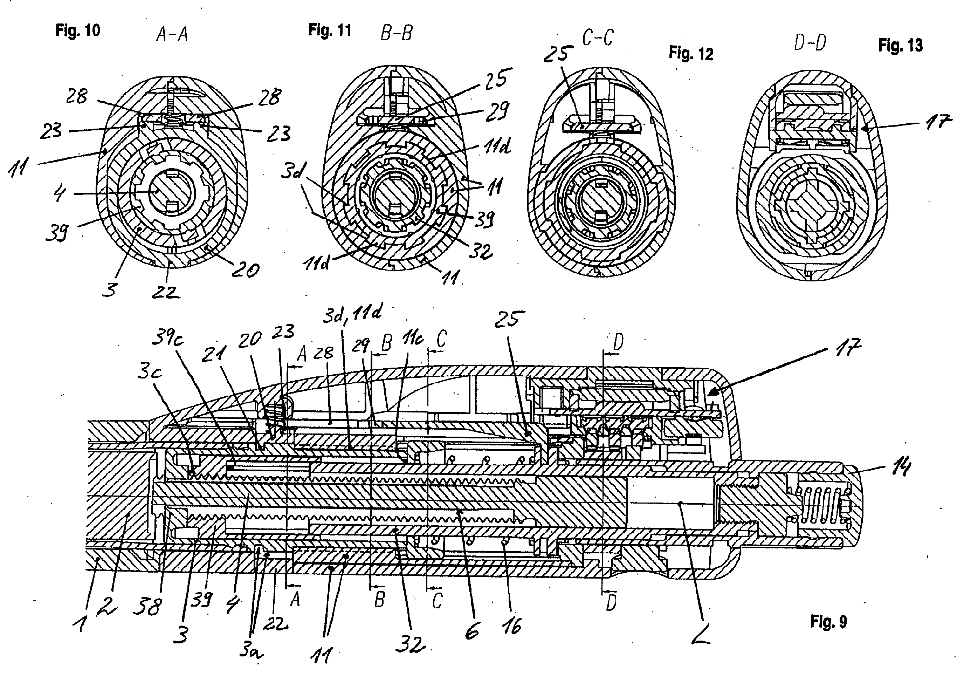

Die Figuren 9 bis 13 zeigen in einem Längsschnitt und vier Querschnitten ein zweites Ausführungsbeispiel eines Injektionsgeräts. Das Injektionsgerät des zweiten Ausführungsbeispiels gleicht demjenigen des ersten Ausführungsbeispiels in Bezug auf die Verriegelung und die Verriegelungssperre 25 derart, dass auf die diesbezügliche Beschreibung des ersten Ausführungsbeispiels verwiesen wird. Insbesondere ist die Verriegelungssperre 25 des zweiten Ausführungsbeispiels in Bezug auf alle funktionalen Details mit derjenigen des ersten Ausführungsbeispiels identisch. Das gleiche gilt für die Verriegelungselemente 3a und 21.

Der Verriegelungsring 20 und die Lage der Sperrnocken 23 relativ zu dem Verriegelungselement 21 und relativ zu der Verriegelungssperre 25 in dem Ausgangszustand des Geräts sind besonders gut in den Querschnitten der Figuren 10, 11 und 12 zu erkennen, auf die diesbezüglich auch stellvertretend für das erste Ausführungsbeispiel verwiesen wird.

Das Injektionsgerät des zweiten Ausführungsbeispiels unterscheidet sich von dem ersten Ausführungsbeispiel durch den Eingriff und den Bewegungsablauf der an der Dosierung beteiligten Komponenten. Ferner erfüllt der Mechanikhalter über die Funktionen des Mechanikhalters des ersten Ausführungsbeispiels hinaus insbesondere die Funktion der Positionierung des Dosiseinstellglieds in diskreten Drehwinkelpositionen, die relativ zu dem Mechanikhalter zum Zwecke der Dosierung veränderbar sind. Die Blockiereinrichtung des zweiten Ausführungsbeispiels ist hingegen einfacher ausgeführt als die des ersten Ausführungsbeispiels. Im Folgenden werden in erster Linie nur die Unterschiede im Vergleich zum ersten Ausführungsbeispiel beschrieben, wobei für Komponenten, die ihrer grundsätzlichen Funktion nach den gleichnamigen Komponenten des ersten Ausführungsbeispiels gleichen, aber in Details unterscheiden, 30iger Zahlen mit gleicher Endziffer oder exakt die gleichen Bezugszeichen, wie bei dem ersten Ausführungsbeispiel, verwendet werden. Soweit zum zweiten Ausführungsbeispiel keine Ausführungen gemacht werden, sollen die entsprechenden Ausführungen zum ersten Ausführungsbeispiel gelten.

In dem zweiten Ausführungsbeispiel ist das relativ zu dem hinteren Gehäuseabschnitt 11 axial linear bewegbare und um die Längsachse L verdrehbare Dosier- und Betätigungselement 32 mit dem Dosiseinstellglied 39 verdrehgesichert verbunden. Das Dosier- und Betätigungselement 32 und das Dosiseinstellglied 39 sind relativ zueinander und relativ zu den Gehäuseabschnitten 1, 3 und 11 in und gegen die Vorschubrichtung bewegbar. Die Kolbenstange 4 wird von dem Mechanikhalter 3 verdrehgesichert gehalten. Die mit dem ersten Ausführungsbeispiel funktionsgleiche Rückzugsperreinrichtung 6 verhindert im Zusammenwirken mit Sperrelementen der einstückig an dem Mechanikhalter 3 geformten Blockiereinrichtung 38 eine Bewegung der Kolbenstange 4 entgegen der Vorschubrichtung, lässt eine Bewegung in die Vorschubrichtung jedoch zu. Die Sperrelemente bilden gleichzeitig die Rückzugsperre und die Verdrehsicherung für die Kolbenstange 4. Des Weiteren bildet wie bereits im ersten Ausführungsbeispiel das Dosier- und Betätigungselement 32 eine Gleitführung für die Kolbenstange 4.

Bei der Dosierung führt das Dosier- und Betätigungselement 32 die gleiche rotatorische Dosierbewegung wie das Dosier- und Betätigungselement 12 des ersten Ausführungsbeispiels aus. Allerdings wird aufgrund des verdrehgesicherten Eingriffs das Dosiseinstellglied 39 bei der rotatorischen Dosierbewegung mitgenommen. Der Gewindeeingriff zwischen der Kolbenstange 4 und dem Dosiseinstellglied 39 ist wieder mit demjenigen des ersten Ausführungsbeispiels vergleichbar, so dass ein von dem Dosiseinstellglied 39 gebildeter Anschlag 39c aufgrund der rotatorischen Dosierbewegung und des Gewindeeingriffs mit der Kolbenstange 4 im Zuge der Dosierung entgegen der Vorschubrichtung auf ein vorderes Ende des Dosier- und Betätigungselements 32 zu bewegt wird. Im Gegensatz zum ersten Ausführungsbeispiel vollführt das Dosiseinstellglied 39 somit bei der Dosierung eine rotatorische Dosierbewegung und eine translatorische Dosierbewegung relativ zu dem vorderen Gehäuseabschnitt, während die Kolbenstange 4 stillsteht. Durch die nach Abschluss der Dosierung erfolgende Ausschüttbewegung des Dosier- und Betätigungselements 32 wird die Kolbenstange 4 um die Weglänge vorgeschoben, die dem durch die Dosierung eingestellten lichten Abstand zwischen einer Anschlagfläche des Dosiseinstellglieds 39 und dem Ausschüttanschlag 3c des Mechanikhalters 3 entspricht.

Die translatorische Dosierbewegung des Dosiseinstellglieds 39 wird gegen die Vorschubrichtung von einem hinteren Translationsanschlag 11 c begrenzt, den der hintere Gehäuseabschnitt 11 selbst unmittelbar bildet. Auch bei dem zweiten Ausführungsbeispiel bildet die Längsachse L die Rotations- und Translationsachse der Komponenten, die an der Dosierung und Produktausschüttung beteiligt sind.

Der vordere Gehäuseabschnitt 1, 3 bildet wie bei dem ersten Ausführungsbeispiel eine Gleitführung für das Dosiseinstellglied 39. Zur Ausbildung der Gleitführung stehen eine innere Mantelfläche des Mechanikhalters 3 und eine äußere Mantelfläche des Dosiseinstellglieds 39 miteinander in Gleitkontakt. Das Dosier- und Betätigungselement 32 steht mit einer Innenmantelfläche des Dosiseinstellglieds 39 in einem Eingriff, um die verdrehgesicherte Verbindung zwischen dem Dosiseinstellglied 39 und dem Dosier- und Betätigungselement 32 zu bilden.

In dem zweiten Ausführungsbeispiel weist die Kolbenstange 4 über die Rückzugssperreinrichtung 6 hinaus keine eigene Bremseinrichtung auf. Die Vorderseiten der Sägezähnen der Rückzugssperreinrichtung 6 bilden vielmehr alleine auch die Bremseinrichtung. Die Kolbenstange 4 des zweiten Ausführungsbeispiels kann jedoch durch die Kolbenstange des ersten Ausführungsbeispiels ersetzt werden. Entsprechend wären in diesem Falle durch den Mechanikhalter 3 des zweiten Ausführungsbeispiels auch wenigstens ein Bremselement, vorzugsweise beide Bremselemente des ersten Ausführungsbeispiels auszubilden.

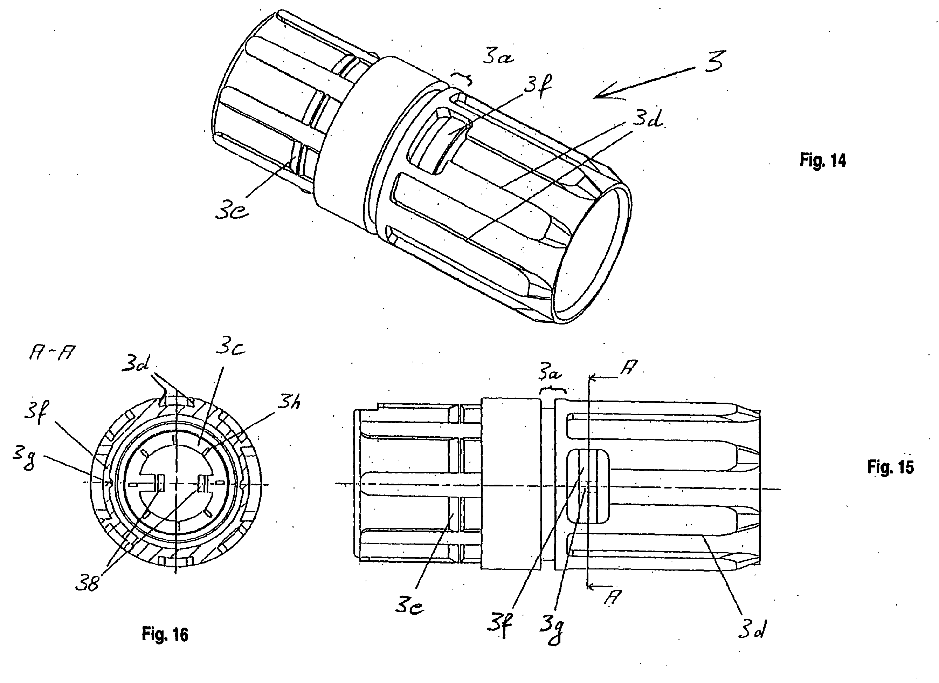

Die Figuren 14 bis 16 zeigen den Mechanikhalter 3 des zweiten Ausführungsbeispiels in einer perspektivischen Darstellung, einer Seitenansicht und in dem in der Seitenansicht eingetragenen Querschnitt A-A. Der Mechanikhalter 3 ist wie beim ersten Ausführungsbeispiel als einteiliges Hülsenteil ausgeführt, vorzugsweise als Kunststoffspritzgussteil. Er weist an dem Außenmantel eines vorderen Hülsenabschnitts einen Wulst 3e auf. Der vordere Hülsenabschnitt wird in das Reservoirteil 1 eingesteckt und mittels des Wulstes 3e zumindest für den Verwender unlösbar mit dem Reservoirteil 1 verrastet.

Das Verriegelungselement 3a ist an einem mittleren Hülsenabschnitt des Mechanikhalters 3 wie bei dem ersten Ausführungsbeispiel ausgebildet.

Ein hinterer Hülsenabschnitt, der sich an das Verriegelungselement 3a anschließt, bildet an seinem Außenumfang eine Mehrzahl von Axialführungen 3d. Die Axialführungen 3d werden von Führungsrippen gebildet, die an dem Außenumfang des hinteren Hülsenabschnitts radial aufragen. Genauer gesagt wird die Axialführung von den axial sich erstreckenden, geraden Seitenwänden dieser Führungsrippen gebildet, so dass, wie bei dem ersten Ausführungsbeispiel, axiale Führungskanäle erhalten werden. Die Führungsrippen ragen von dem mittleren Hülsenabschnitt aus wie Finger bis an das hintere Ende des Mechanikhalters 3, wo sie axial verjüngt auslaufen. Die Axialführung 3d dient der Linearführung des hinteren Gehäuseabschnitts 11 bei dem Verbinden des Reservoirmoduls 10 mit dem Dosier- und Betätigungsmodul 30. Wie in Figur 9 und am besten in Figur 11 zu erkennen ist ragen von einer Innenmantelfläche des hinteren Gehäuseabschnitts 11 der Anzahl nach entsprechend und der Form nach angepasst Eingriffselemente 11d radial einwärts ab. In jede der Axialführungen 3d ragt ein Eingriffselement 11d ein und wird von der Axialführung 3d linear geführt, wenn der vordere Gehäuseabschnitt 1, 3 und der hintere Gehäuseabschnitt 11 zum Verbinden ineinander geschoben werden. Auf diese Weise wird sichergestellt, dass zwischen dem vorderen Gehäuseabschnitt 1, 3 und dem hinteren Gehäuseabschnitt 11 keine Relativdrehung stattfinden kann, wenn im Zuge des Verbindens der verdrehgesicherte Eingriff zwischen dem Dosier- und Betätigungselement 32 und dem Dosiseinstellglied 39 hergestellt wird.

Indem die Führungsrippen an ihren hinteren Enden axial verjüngt auslaufen und die Führungskanäle so zu Einführtrichtern verbreitert sind, wird eine Zentrierung zwischen dem vorderen Gehäuseabschnitt 1, 3 und dem hinteren Gehäuseabschnitt 11 zum Zwecke des Verbindens erleichtert. Die Führungsrippen laufen an ihren Enden auch radial zur Mantelfläche des Mechanikhalters 3 verjüngt aus, wodurch die Zentrierung der Gehäuseabschnitte 1, 3 und 11 in eine durch die Axialführung 3d vorgegebene Drehwinkelposition relativ zueinander noch mehr erleichtert wird.

Ebenso wie bei dem Ineinanderschieben der vordere Gehäuseabschnitt 1, 3 und der hintere Gehäuseabschnitt 11 an einer Relativdrehung zueinander gehindert werden, wird auch das Dosiseinstellglied 39 in Bezug auf seine Drehwinkelposition relativ zu dem vorderen Gehäuseabschnitt 1, 3 fixiert, wobei diese Fixierung lösbar ist, um die für die Dosierung erforderliche Drehbewegung des Dosiseinstellglieds 39 zuzulassen. Um daher zum einen die Dosierbewegung des Dosiseinstellglieds 39 zu ermöglichen, aber zum anderen eine unerwünschte Dosierbewegung durch das Herstellen der Verbindung zwischen dem vorderen Gehäuseabschnitt 1, 3 und dem hinteren Gehäuseabschnitt 11 zu verhindern, wird das Dosiseinstellglied 39 von dem Mechanikhalter 3 mittels einer lösbaren Rastverbindung in diskreten Drehwinkelpositionen fixiert.

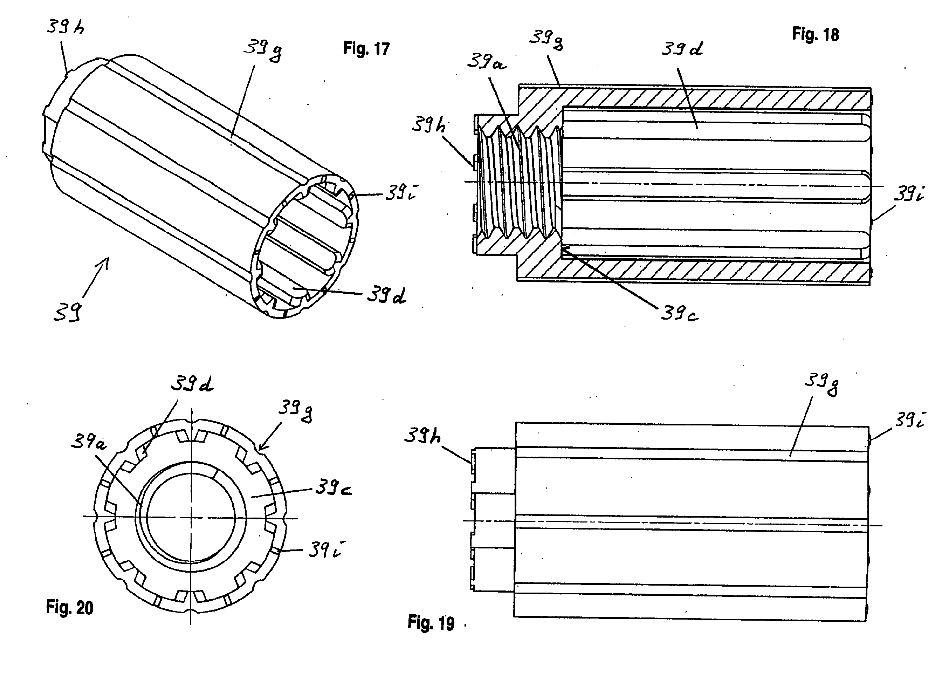

Die Figuren 17 bis 20 zeigen das Dosiseinstellglied 39 in Einzeldarstellungen. Für die Ausbildung der Rastverbindung sind an der Außenmantelfläche des Dosiseinstellglieds 39 über den Umfang in regelmäßiger Teilung verteilt mehrere Rastaufnahmen 39g ausgebildet. Jede der Rastaufnahmen 39g wird von einer geraden, axial sich erstreckenden Rinne mit einer im Querschnitt gerundet verlaufenden Kontur gebildet.

Der Mechanikhalter 3 ist mit zwei Rastnasen 3g (Figur 15 und 16) versehen. Die beiden Rastnasen 3g ragen in dem hinteren Hülsenabschnitt des Mechanikhalters 3 von einer Innenmantelfläche des Mechanikhalters 3 radial einwärts ab. Sie sind einander diametral gegenüberliegend angeordnet. Der jeweilige Mantelbereich des Mechanikhalters 3, an dem eine der Rastnasen 3g angeformt ist, bildet ein in radialer Richtung elastisch nachgiebiges Federelement 3f. Aufgrund der elastischen Nachgiebigkeit und der gerundeten Form der Rastnasen 3g in Verbindung mit dem gerundeten Profil der Rastaufnahmen 39g ist der Rasteingriff der Rastnasen 3g in die gegenüberliegenden Rastaufnahmen 39g lösbar. Die Lösbarkeit ist für die Dosisauswahl erforderlich. Andererseits ist der Rasteingriff jedoch so gestaltet, dass die Drehwinkelfixierung des Dosiseinstellglieds 39 so stabil ist, dass bei dem Verbinden des vorderen Gehäuseabschnitts 1, 3 mit dem hinteren Gehäuseabschnitt 11 keine unerwünschte Dosierbewegung des Dosiseinstellglieds 39 stattfinden kann, wenn die Drehkopplung des Dosier- und Betätigungselements 32 mit dem Dosier- und Betätigungselement 32 hergestellt wird. Die Rastverbindung zwischen dem Mechanikhalter 3 und dem Dosiseinstellglied 39 ergibt als vorteilhaften Nebeneffekt ein taktiles Signal bei der Dosierung. Um die günstige Elastizität des Federelements 3f zu erhalten, ist der hintere Hülsenabschnitt des Mechanikhalters 3 im betreffenden Mantelbereich ausgeschnitten, so dass das Federelement 3f als ein im Umfangsrichtung sich erstreckendes Ringsegment erhalten wird, das axial beidseits freiliegt.

Aus den Figuren 17, 18 und 20 ebenfalls erkennbar sind Axialführungen 39d für den verdrehsicheren Eingriff des Dosiseinstellglieds 39 mit dem Dosier- und Betätigungselement 32. Das Dosier- und Betätigungselement 32 ist mit wenigstens einem Eingriffselement versehen, um die axiale Linearführung, d.h. die Verdrehsicherung, zwischen dem Dosier- und Betätigungselement 32 und dem Dosiseinstellglied 39 zu erhalten. Die Axialführungen 39d sind wieder Führungskanäle, die von mehreren, axial sich gerade erstreckenden Führungsrippen gebildet werden. Jede der Führungsrippen läuft an ihrem hinteren, dem Dosier- und Betätigungselement 32 zugewandten Ende axial und radial verjüngt aus, um die Zentrierung zwischen dem Dosier- und Betätigungselement 32 und dem Dosiseinstellglied 39 bei der Herstellung des verdrehsicheren Eingriffs zu erleichtern. Für die axiale Linearführung von Dosiseinstellglied 39 und Dosier- und Betätigungselement 32 wird somit die gleiche Konstruktion wie für die axiale Linearführung der Gehäuseabschnitte 1, 3 und 11 verwendet.

Der Vollständigkeit wegen sei schließlich auch auf das Dosiergewinde 39a und den Ausschüttanschlag 39c des Dosiseinstellglieds 39 hingewiesen, die am besten in Figur 18 zu erkennen sind.

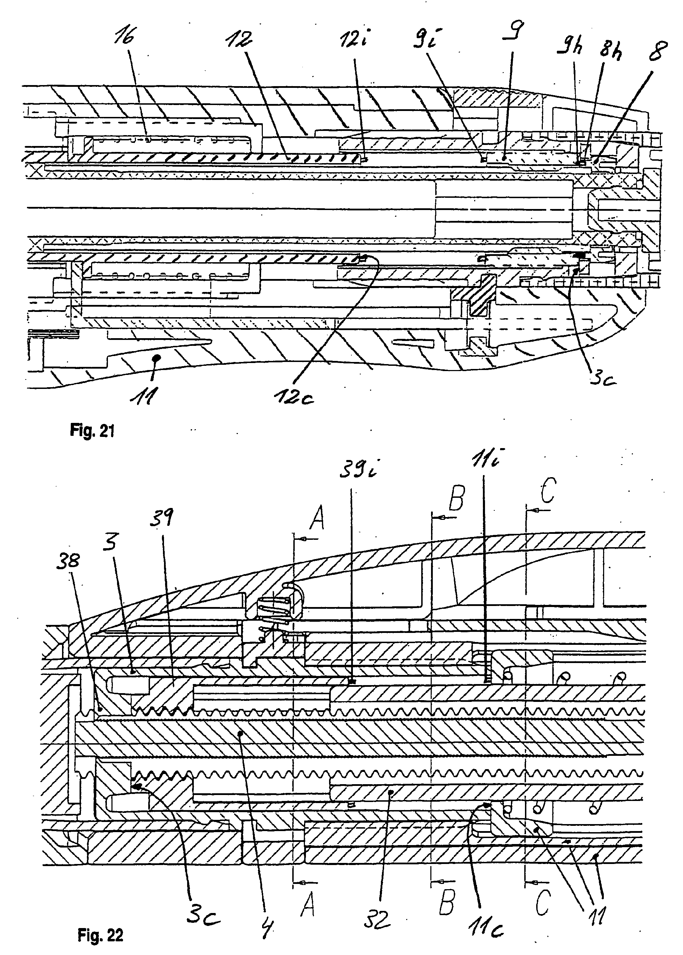

Schließlich sind für das Dosiseinstellglied 39 zwei Verdrehsicherungen vorgesehen, die in den beiden axialen Endpositionen des Dosiseinstellglieds 39 wirksam werden. Diesbezüglich sei ergänzend auf Figur 22 hingewiesen.

Um zu verhindern, dass die Kolbenstange 4 in Reaktion auf eine rotatorische Dosierbewegung des Dosiseinstellglieds 39 zurückbewegt werden könnte, sind an einem vorderen Ende des Dosiseinstellglieds 39 Verdrehanschläge 39h geformt. Die Verdrehanschläge 39h stehen in der vorderen Endposition, die das Dosiseinstellglied 39 unmittelbar nach einer Produktausschüttung bzw. vor einer Dosisauswahl einnimmt, mit Verdrehgegenanschlägen 3h in Eingriff, die an dem Mechanikhalter 3 angeformt sind (Fig. 16). Die Verdrehanschläge 39h ragen von einer vorderen Stirnseite des Dosiseinstellglieds 39 axial ab, und die Verdrehgegenanschläge 3h ragen von einer axial zugewandten Stirnfläche des Mechanikhalters 3, die den Aufschüttanschlag 3c bildet, den Verdrehanschlägen 39h axial entgegen. Der Eingriff zwischen den Verdrehanschlägen 39h und den Verdrehgegenanschlägen 3h ist derart, dass er eine rotatorische Dosierbewegung in eine Drehrichtung zulässt, die eine von dem Ausschüttanschlag 3c weg gerichtete translatorische Dosierbewegung des Dosiseinstellglieds 39 bewirkt, aber in der vorderen axialen Endposition eine rotatorische Dosierbewegung in die Gegendrehrichtung verhindert.

Ferner ist ein weiteres Paar von Verdrehanschlägen und Verdrehgegenanschlägen vorgesehen, die in grundsätzlich gleicher Weise wie die Anschläge 3h und 39h geformt sind und zusammenwirken. Bei diesem zweiten Verdrehanschlagpaar handelt es sich zum einen um Verdrehanschläge 39i, die von einer hinteren Stirnfläche des Dosiseinstellglieds 39 axial abragen, und zum anderen um Verdrehgegenanschläge 11i, die von der zugewandten Anschlagstirnfläche des hinteren Translationsanschlags 11c axial auf das Dosiseinstellglied 39 zuragen, in der Figur 9 aufgrund ihrer geringen Abmessungen jedoch nicht erkennbar sind. Durch das hintere Verdrehanschlagpaar 11i/39i wird in der hinteren Endposition verhindert, dass die Kolbenstange 4 in Reaktion auf eine gegen die Anschlagstirnfläche des hinteren Translationsanschlags 11c gerichtete Dosierbewegung des Dosiseinstellglieds in Vorschubrichtung bewegt werden kann.
Die Höhe, d.h. die axiale Länge, sämtlicher Verdrehanschläge 3h, 39h, 11i und 39i ist auf die Gewindesteigung der im Eingriff befindlichen Dosiergewinde der Kolbenstange 4 und des Dosiseinstellglieds 39 abgestimmt. Die Verdrehanschläge sind axial so kurz, dass diejenige rotatorische Dosierbewegung nicht behindert wird, durch die das Dosiseinstellglied 39 von dem jeweiligen Translationsanschlag 3c oder 11c wegbewegt wird.

Bei der Montage der Komponenten des Reservoirmoduls 10 wird das Dosiseinstellglied 39 bis in eine vorgegebene Axialposition, wie sie aus Figur 9 ersichtlich ist, auf die Kolbenstange 4 aufgeschraubt. Anschließend wird die Kolbenstange 4 mit dem aufgeschraubten Dosiseinstellglied 39 von hinten in den Mechanikhalter 3 eingeführt, bis dessen Blockiereinrichtung 38 in Sperreingriff mit der Rückzugssperreinrichtung 6 der Kolbenstange 4 gelangt und ferner der verdrehgesicherte Eingriff zwischen den Verdrehanschlägen 39h des Dosiseinstellglieds 39 und den Verdrehgegenanschlägen 3h des Mechanikhalters 3 hergestellt ist. Bereits während der Einführung in den Mechanikhalter 3 wird das Dosiseinstellglied 39 in einer Drehwinkelrastposition durch den Rasteingriff zwischen den Rastnasen 3g und den Rastaufnahmen 39g von dem Mechanikhalter 3 axial linear geführt, bis das Dosiseinstellglied 39 an den Ausschüttanschlag 3c des Mechanikhalters 3 anstößt. In dieser vorderen Endposition des Dosiseinstellglieds 39 relativ zu dem Mechanikhalter 3 ist gleichzeitig auch bereits der verdrehsichere Eingriff zwischen den Verdrehanschlägen 3h und 39h hergestellt.

In diesem Zustand werden der Mechanikhalter 3 und ein bereits mit einem Reservoir ausgestattetes Reservoirteil 1 miteinander verbunden.

In einem nächsten Schritt wird der hintere Gehäuseabschnitt 11 des komplett montierten Dosier- und Betätigungsmoduls 30 auf den Mechanikhalter 3 geschoben, wobei sich der Mechanikhalter 3 und der hintere Gehäuseabschnitt 11 aufgrund der Axialführungen 3d und der Eingriffselemente 11d des hinteren Gehäuseabschnitts 11 zueinander zentrieren können und nach der Zentrierung aufgrund des Führungseingriffs axial aneinander linear geführt werden. Im Zuge des Aufschiebens gelangt das Dosier- und Betätigungselement 32 in den verdrehgesicherten Eingriff mit dem Dosiseinstellglied 39, wobei auch hier durch eine den Axialführungen 3d und Eingriffselementen 11d entsprechende Linearführung zuerst eine gewisse Zentrierung stattfinden kann.

Das Dosier- und Betätigungselement 32 ist in diskreten Drehwinkelrastpositionen mit dem hinteren Gehäuseabschnitt in einem Rasteingriff und wird in dem Rasteingriff, d.h. in der jeweiligen Drehwinkelrastposition, axial linear geführt. Die Drehwinkeldifferenz zwischen zwei aufeinanderfolgenden Drehwinkelrastpositionen entspricht einer Dosiseinheit. Die Linearführung zwischen dem Mechanikhalter 3 und dem hinteren Gehäuseabschnitt 11 einerseits und die diskreten Drehwinkelpositionen des Dosiseinstellglieds 39 relativ zu dem Mechanikhalter 3 (Rastnasen 3g und Rastaufnahmen 39g) und die Drehwinkelrastpositionen des Dosier- und Betätigungselements 32 relativ zu dem hinteren Gehäuseabschnitt 11 andererseits sind aufeinander so abgestimmt, dass ein lineares Übereinanderschieben der beiden Gehäuseabschnitte 1, 3 und 11 stets in solch einer Drehwinkelposition stattfindet, dass auch das Dosiseinstellglied 39 und das Dosier- und Betätigungselement 32 für ihren verdrehgesicherten Eingriff relativ zueinander ausgerichtet sind, so dass während des Verbindens des Reservoirmoduls 10 mit dem Dosier- und Betätigungsmodul 30 keine Relativdrehung zwischen den an der Dosierung beteiligten Komponenten stattfindet.

In Bezug auf die weiteren Details der Montage, insbesondere der Herstellung des Verriegelungseingriffs, und zur Funktionsweise des Injektionsgeräts nach dem zweiten Ausführungsbeispiel wird auf die Beschreibung zu dem ersten Ausführungsbeispiel verwiesen.

Auch bei dem Injekionsgerät nach dem ersten Ausführungsbeispiel können Verdrehsicherungen vorgesehen sein, die in den beiden axialen Endpositionen des Dosiseinstellglieds 9 des ersten Ausführungsbeispiels unerwünschte Reaktionsbewegungen der Kolbenstange 4 verhindern. Figur 21 zeigt die beiden Verdrehsicherungen, die in gleicher Weise wie die Verdrehsicherungen des zweiten Ausführungsbeispiels geformt sind. Allerdings werden die bei dem zweiten Ausführungsbeispiel an den Gehäuseabschnitten 1, 3 und 11 geformten Verdrehgegenanschläge bei dem ersten Ausführungsbeispiel zum einen von der Blockiereinrichtung 8 und zum anderen von dem Dosier- und Betätigungselement 12 gebildet. So sind an der Stirnfläche der Blockiereinrichtung 8, die dem Dosiseinstellglied 9 axial zugewandt ist, mehrere Verdrehanschläge 8h angeformt, die auf das Dosiseinstellglied 9 axial zu ragen. Da die Blockiereinrichtung 8 von dem vorderen Gehäuseabschnitt 1, 3 axial unbeweglich gelagert wird und mit der Kolbenstange 4 verdrehgesichert verbunden ist, wird auch durch das vordere Verdrehanschlagpaar 8h/9h eine Verdrehsicherung für die zwischen der Kolbenstange 4 und dem Dosiseinstellglied 9 stattfindende rotatorische Dosierbewegung erhalten. Das zweite Verdrehanschlagpaar ist zwischen dem Dosiseinstellglied 9 und dem hinteren Translationsanschlag 12c gebildet. Wie bei dem zweiten Ausführungsbeispiel ragen von der Stirnfläche des Translationsanschlags 12c, die dem Dosiseinstellglied 9 axial zugewandt ist, mehrere Verdrehanschläge 12i axial in Richtung auf das Dosiseinstellglied 9 vor. Das Dosiseinstellglied 9 ist wie bei dem zweiten Ausführungsbeispiel an seiner Rückseite mit Verdrehanschlägen 9i versehen, die in der hinteren axialen Endposition des Dosiseinstellglieds 9 in Eingriff mit den Verdrehanschlägen 12i gelangen. In der hinteren axialen Endposition des Dosiseinstellglieds 9 wird durch das hintere Verdrehanschlagpaar 9i/12i nur diejenige rotatorische Dosierbewegung zugelassen, die eine translatorische Dosierbewegung des Dosiseinstellglieds 9 in die Vorschubrichtung bewirkt.

### Bezugszeichen:

- 1: Reservoirteil, Ampullenhalter
- 2: Reservoir, Ampulle
- 3: Mechanikhalter
- 3a: Erstes Verriegelungselement
- 3b: Fixiereinrichtung
- 3c: Ausschüttanschlag, Translationsanschlag
- 3d: Axialführung
- 3e: Wulst
- 3f: Federelement
- 3g: Rastnase
- 3h: Verdrehanschlag
- 4: Kolbenstange
- 4a: Verbindungsabschnitt
- 5: Gewindeabschnitt
- 6: Rückzugsperreinrichtung, Zahnreihe
- 7: Vorschubbremseinrichtung, Zahnreihe
- 8: Blockiereinrichtung
- 8a: Sperrelement
- 8b: Bremselement
- 8h: Verdrehanschlag
- 9: Dosiseinstellglied
- 9h: Verdrehanschlag
- 9i: Verdrehanschlag
- 10: Reservoirmodul
- 11: hinterer Gehäuseabschnitt
- 11d: Eingriffselement
- 11i: Verdrehanschlag
- 12: Dosier- und Betätigungselement
- 12i: Verdrehanschlag
- 13: Dosiermitnehmer
- 14: Verschluss
- 15a: Mitnehmer, Ringsteg
- 15b: Mitnehmer, Ringsteg
- 16: Rückstelleinrichtung
- 17: Zähl- und Anzeigeeinrichtung
- 18: -
- 19: -
- 20: Verriegelungsring
- 21: Zweites Verriegelungselement
- 22: Entriegelungsknopf
- 23: Sperrnocken
- 24: Rückstelleinrichtung
- 25: Verriegelungssperre
- 26: Mitnehmer, Steg
- 27: Axialausnehmung
- 28: Sperrzunge
- 29: Einrückausnehmung
- 30: Dosier- und Betätigungsmodul
- 31: Anschlagelement
- 32: Dosier- und Betätigungselement
- 33-37: -
- 38: Blockiereinrichtung
- 39: Dosiseinstellglied
- 39a: Dosiergewinde
- 39c: Ausschüttanschlag
- 39d: Axialführung
- 39g: Rastaufnahme, Axialführung
- 39h: Verdrehanschlag
- 39i: Verdrehanschlag

## Patentansprüche

1. Verabreichungsgerät, umfassend:
a) einen vorderen Gehäuseabschnitt (1, 3) mit einem Reservoir (2) für ein ausschüttbares Produkt und einem ersten Verriegelungselement (3a),
b) einen Kolben, der in dem Reservoir (2) in eine Vorschubrichtung auf einen Reservoirauslass zu verschiebbar aufgenommen ist, um Produkt auszuschütten,
c) eine Kolbenstange (4),
d) einen hinteren Gehäuseabschnitt (11), der mit dem vorderen Gehäuseabschnitt (1, 3) lösbar verbunden ist und ein zweites Verriegelungselement (21) aufweist, das mit dem ersten Verriegelungselement (3a) in einem Verriegelungseingriff steht, und
e) ein von dem hinteren Gehäuseabschnitt (11) in und gegen die Vorschubrichtung bewegbar gelagertes Antriebselement (12; 32), das bei einer Ausschüttbewegung in die Vorschubrichtung auf die Kolbenstange (4) wirkt, um den Kolben in Vorschubrichtung zu bewegen,
**gekennzeichnet durch**
f) eine Verriegelungssperre (25) für die Verriegelungselemente (3a, 21), die mit dem Antriebselement (12; 32) so gekoppelt ist, das der Verriegelungseingriff nur in einer Freigabestellung des Antriebselements (12; 32) gelöst werden kann.

2. Verabreichungsgerät nach Anspruch 1, **dadurch gekennzeichnet, dass** wenigstens eines der Verriegelungselemente (3a; 21) in eine Richtung quer zu der Vorschubrichtung bewegbar mit seinem zugeordneten Gehäuseabschnitt (1, 3; 11) verbunden ist, um den Verriegelungseingriff herzustellen und zu lösen.

3. Verabreichungsgerät nach dem vorhergehenden Anspruch, **dadurch gekennzeichnet, dass** das wenigstens eine bewegbare Verriegelungselement (3a; 21) in eine Richtung radial zu einer Längsachse (L) des Geräts bewegbar ist.

4. Verabreichungsgerät nach einem der zwei vorhergehenden Ansprüche, **dadurch gekennzeichnet, dass** das wenigstens eine bewegbare Verriegelungselement (3a; 21) betätigt werden muss, um den Verriegelungseingriff in der Freigabestellung des Antriebselements (12; 32) zu lösen.

5. Verabreichungsgerät nach dem vorhergehenden Anspruch, **dadurch gekennzeichnet, dass** das wenigstens eine bewegbare Verriegelungselement (3a; 21) mit einem Entriegelungsknopf (22) verbunden ist.

6. Verabreichungsgerät nach einem der vorhergehenden Ansprüche, **dadurch gekennzeichnet, dass** der vordere Gehäuseabschnitt (1, 3) und der hintere Gehäuseabschnitt (11) durch die in dem Verriegelungseingriff befindlichen Verriegelungselemente (3a; 21) in und gegen die Vorschubrichtung aneinander festgelegt sind.

7. Verabreichungsgerät nach einem der vorhergehenden Ansprüche, **dadurch gekennzeichnet, dass** das erste Verriegelungselement (3a) ein fester Bestandteil des vorderen Gehäuseabschnitts (1, 3) oder das zweite Verriegelungselement ein fester Bestandteil des hinteren Gehäuseabschnitts (11) ist.

8. Verabreichungsgerät nach einem der vorhergehenden Ansprüche, **dadurch gekennzeichnet, dass** eines der Verriegelungselemente (3a; 21) an seinem zugeordneten Gehäuseabschnitt (1, 3; 11) in und aus dem Verriegelungseingriff bewegbar angeordnet ist und eine Rückstelleinrichtung (24) elastisch auf das bewegbar angeordnete Verriegelungselement (21) wirkt, um ein selbsttätiges Lösen des Verriegelungseingriffs zu verhindern.

9. Verabreichungsgerät nach einem der vorhergehenden Ansprüche, **dadurch gekennzeichnet, dass** ein Sperrschieber, der die Verriegelungssperre (25) mitbildet oder bereits alleine bildet, und das Antriebselement (12; 32) so gekoppelt sind, dass das Antriebselement (12; 32) den Sperrschieber (25) bei der Ausschüttbewegung mitnimmt.

10. Verabreichungsgerät nach dem vorhergehenden Anspruch, **dadurch gekennzeichnet, dass** der Sperrschieber (25) quer zu einer Richtung bewegbar ist, in die eines der Verriegelungselemente (3a; 21) bewegt werden muss, um den Verriegelungseingriff zu lösen.

11. Verabreichungsgerät nach einem der zwei vorhergehenden Ansprüche, **dadurch gekennzeichnet, dass** das Betätigungselement (12) und der Sperrschieber (25) in und gegen die Vorschubrichtung relativ zueinander nicht bewegbar sind.

12. Verabreichungsgerät nach einem der drei vorhergehenden Ansprüche, **dadurch gekennzeichnet, dass** der Sperrschieber (25) wenigstens eine Einrückausnehmung (29) aufweist, die nur in der Freigabestellung des Antriebselements (12; 32) mit einer Verriegelungseinrichtung (20), die eines der Verriegelungselemente (3a; 21) bildet, so in Überdeckung ist, dass die Verriegelungseinrichtung (20) zumindest teilweise in die Einrückausnehmung (29) bewegbar ist, um den Verriegelungseingriff zu lösen.

13. Verabreichungsgerät nach einem der vorhergehenden Ansprüche, **dadurch gekennzeichnet, dass** das Verabreichungsgerät ein Dosiseinstellglied (9; 39) umfasst, das mit der Kolbenstange (4) in einem Dosiereingriff steht, so dass es bei einer Dosierbewegung für eine Auswahl einer Produktdosis relativ zu der Kolbenstange (4) und bei einer Ausschüttbewegung für eine Produktausschüttung gemeinsam mit der Kolbenstange (4) relativ zu dem vorderen Gehäuseabschnitt (1, 3) in die Vorschubrichtung bewegt wird.

14. Verabreichungsgerät nach einem der vorhergehenden Ansprüche, **dadurch gekennzeichnet, dass** das Verabreichungsgerät ein Dosiseinstellglied (9; 39) umfasst, das mit der Kolbenstange (4) in solch einem Eingriff steht, das es für eine Auswahl einer Produktdosis relativ zu dem vorderen Gehäuseabschnitt (1, 3) und der Kolbenstange (4) gegen die Vorschubrichtung und für eine Ausschüttung der Produktdosis gemeinsam mit der Kolbenstange (4) relativ zu dem vorderen Gehäuseabschnitt (1, 3) in die Vorschubrichtung bewegbar ist.

15. Verabreichungsgerät nach einem der zwei vorhergehenden Ansprüche, **dadurch gekennzeichnet, dass** das Dosiseinstellglied (9; 39) in Vorschubrichtung gegen einen Ausschüttanschlag (3c) des vorderen Gehäuseabschnitts (1, 3) stößt, der die Ausschüttbewegung in Vorschubrichtung begrenzt.

16. Verabreichungsgerät nach einem der drei vorhergehenden Ansprüche, **dadurch gekennzeichnet, dass** das Antriebselement (12; 32) bei seiner Ausschüttbewegung gegen das Dosiseinstellglied (9; 39) drückt und dadurch die Kolbenstange (4) mitnimmt.

17. Verabreichungsgerät nach einem der vorhergehenden Ansprüche, **dadurch gekennzeichnet, dass** das Antriebselement (12; 32) von dem hinteren Gehäuseabschnitt (11) auch zur Ausübung einer Dosierbewegung bewegbar gelagert und mit der Kolbenstange (4) so gekoppelt ist, dass es die Kolbenstange (4) bei der Dosierbewegung mitnimmt.

18. Verabreichungsgerät nach einem der vorhergehenden Ansprüche, **dadurch gekennzeichnet, dass** eine Blockiereinrichtung (8; 38) vorgesehen ist, die mit der Kolbenstange (4) in Eingriff steht, um eine Bewegung der Kolbenstange (4) entgegen der Vorschubrichtung zu verhindern.

19. Verabreichungsgerät nach einem der vorhergehenden Ansprüche, **dadurch gekennzeichnet, dass** eine Blockiereinrichtung (8) vorgesehen ist, die mit der Kolbenstange (4) in einem Bremseingriff steht, der die Bewegung der Kolbenstange (4) in die Vorschubrichtung erschwert.

20. Verabreichungsgerät nach einem der vorhergehenden Ansprüche, **dadurch gekennzeichnet, dass** das der vordere Gehäuseabschnitt (1, 3) ein das Reservoir (2) enthaltenden Reservoirteil (1) und einen Mechanikhalter (3) umfasst, der mit dem Reservoirteil (1) in Bezug auf die Vorschubrichtung nicht bewegbar verbunden ist und die Kolbenstange (4) hält und das erste Verriegelungselement (3a) umfasst.

21. Verabreichungsgerät nach einem der vorhergehenden Ansprüche, **dadurch gekennzeichnet, dass** eine Kanüle von höchstens 30 Gauge, vorzugsweise eine 31 oder 32 Gauge Kanüle, oder eine Kanüle außerhalb der in ISO 9626 spezifizierten Maße mit einem Außendurchmesser von höchstens einer 30 Gauge Kanüle ein infundierendes Teil des Verabreichungsgeräts bildet.

22. Antriebsvorrichtung für das Verabreichungsgerät nach Anspruch 1, die mit einem Reservoirmodul (10) des Verabreichungsgeräts lösbar verbunden werden kann, wobei das Reservoirmodul (10) das Reservoir (2) für ein ausschüttbares Produkt und den in dem Reservoir (2) zur Ausschüttung von Produkt in eine Vorschubrichtung verschiebbar aufgenommenen Kolben aufweist, die Antriebsvorrichtung (30) umfassend:
a) den hinteren Gehäuseabschnitt (11) des Verabreichungsgeräts, der mit dem Reservoirmodul (10) verbindbar ist und das Verriegelungselement (21) zur Herstellung eines Verriegelungseingriffs mit dem Reservoirmodul (10) aufweist,
b) das von dem hinteren Gehäuseabschnitt (11) bewegbar gelagerte Antriebselement (12; 32)
c) und die Verriegelungssperre (25), die auf das Verriegelungselement (20) wirkt und mit dem Antriebselement (12; 32) so gekoppelt ist, dass die Antriebsvorrichtung (30) mit dem Reservoirmodul (10) nur verbunden werden kann, wenn das Antriebselement (12) relativ zu dem hinteren Gehäuseabschnitt (11) eine Freigabestellung bezüglich der Vorschubrichtung einnimmt.

23. Antriebsvorrichtung nach dem vorhergehenden Anspruch, **dadurch gekennzeichnet, dass** der hintere Gehäuseabschnitt und das zweite Verriegelungselement nach einem der Ansprüche 2 bis 20 den Gehäuseabschnitt (11) und das Verriegelungselement (21) der Antriebsvorrichtung (30) bilden und auch das Antriebselement (12; 32) und die Verriegelungssperre (25) der Antriebsvorrichtung (30) wie das Antriebselement und die Verriegelungssperre nach einem der Ansprüche 2 bis 20 ausgebildet sind.

24. Reservoirmodul für ein Verabreichungsgerät, wobei das Reservoirmodul mit einer Antriebsvorrichtung des Verabreichungsgeräts lösbar verbunden werden kann, das Reservoirmodul (10) umfassend:
a) einen vorderen Gehäuseabschnitt (1, 3) des Verabreichungsgeräts mit einem Reservoir (2) für ein ausschüttbares Produkt,
b) einen Kolben, der in dem Reservoir (2) in eine Vorschubrichtung auf einen Reservoirauslass zu verschiebbar aufgenommen ist, um Produkt auszuschütten, und
c) ein Verriegelungselement (3a), das zur Herstellung eines Verriegelungseingriffs mit der Antriebsvorrichtung (30) von dem vorderen Gehäuseabschnitt (1, 3) gebildet oder für ein Herstellen und Lösen des Verriegelungseingriffs bewegbar mit dem vorderen Gehäuseabschnitt (1, 3) verbunden ist,
**gekennzeichnet durch**
d) eine Kolbenstange (4), die von dem vorderen Gehäuseabschnitt (1, 3) gelagert wird.

25. Reservoirmodul nach dem vorhergehenden Anspruch, **dadurch gekennzeichnet, dass** der vordere Gehäuseabschnitt (1, 3) an einem von dem Reservoirauslass abgewandten, hinteren Mantelbereich einen radialen Vorsprung aufweist, der das Verriegelungselement (3a) bildet oder mitbildet.

26. Reservoirmodul nach dem vorhergehenden Anspruch, **dadurch gekennzeichnet, dass** der vordere Gehäuseabschnitt (1, 3) die Kolbenstange (4) nach einem der Ansprüche 1 bis 20 hält.

27. Reservoirmodul nach einem der drei vorhergehenden Ansprüche, **dadurch gekennzeichnet, dass** der vordere Gehäuseabschnitt (1, 3) eine Blockiereinrichtung (8; 38) nach einem der Ansprüche 1 bis 20 bildet oder hält.

28. Reservoirmodul nach einem der vier vorhergehenden Ansprüche, **dadurch gekennzeichnet, dass** der vordere Gehäuseabschnitt (1, 3) den Ausschüttanschlag (3c) nach Anspruch 15 bildet.

29. Reservoirmodul nach einem der fünf vorhergehenden Ansprüche, **dadurch gekennzeichnet, dass** der vordere Gehäuseabschnitt (1, 3) eine Führung, vorzugsweise eine Linearführung, für das Dosiseinstellglied (9; 39) nach einem der vorhergehenden Ansprüche bildet.

## Claims

1. An administering apparatus, comprising:
a) a front casing section (1, 3) comprising a reservoir (2) for a product which can be delivered and a first latching element (3a);
b) a piston which is accommodated in the reservoir (2) such that it can shift in an advancing direction towards a reservoir outlet, in order to deliver product;
c) a piston rod (4);
d) a rear casing section (11) which is detachably connected to the front casing section (1, 3) and comprises a second latching element (21) which is in latching engagement with the first latching element (3a); and
e) a drive element (12; 32) which is mounted by the rear casing section (11) such that it is movable in and counter to the advancing direction and which acts on the piston rod (4) during a delivery movement in the advancing direction, in order to move the piston in the advancing direction;
**characterised by**
f) a latching block (25) for the latching elements (3a, 21) which is coupled to the drive element (12; 32) such that the latching engagement can only be released in a releasing position of the drive element (12; 32).

2. The administering apparatus according to claim 1, **characterised in that** at least one of the latching elements (3a, 21) is connected to its corresponding casing section (1, 3; 11) such that it can move in a direction transverse to the advancing direction, in order to establish and release the latching engagement.

3. The administering apparatus according to the preceding claim, **characterised in that** the at least one movable latching element (3a; 21) can be moved in a radial direction with respect to a longitudinal axis (L) of the apparatus.

4. The administering apparatus according to any one of the preceding two claims, **characterised in that** the at least one movable latching element (3a; 21) has to be activated in order to release the latching engagement in the releasing position of the drive element (12; 32).

5. The administering apparatus according to the preceding claim, **characterised in that** the at least one movable latching element (3a; 21) is connected to an unlatching button (22).

6. The administering apparatus according to any one of the preceding claims, **characterised in that** the front casing section (1, 3) and the rear casing section (11) are fixed to each other in and counter to the advancing direction by the latching elements (3a, 21) in latching engagement.

7. The administering apparatus according to any one of the preceding claims, **characterised in that** the first latching element (3a) is a fixed component of the front casing section (1, 3) or the second latching element (21) is a fixed component of the rear casing section (11).

8. The administering apparatus according to any one of the preceding claims, **characterised in that** one of the latching elements (3a, 21) is arranged on its corresponding casing section (1, 3; 11) such that it can move in and out of latching engagement, and a restoring means (24) acts elastically on the movably arranged latching element (21) in order to prevent the latching engagement from releasing by itself.

9. The administering apparatus according to any one of the preceding claims, **characterised in that** a blocking slider, forming a part of the latching block (25) or forming it itself alone, and the drive element (12; 32) are coupled such that the drive element (12; 32) slaves the blocking slider (25) during the delivery movement.

10. The administering apparatus according to the preceding claim, **characterised in that** the blocking slider (25) can be moved transverse to a direction in which one of the latching elements (3a; 21) has to be moved in order to release the latching engagement.

11. The administering apparatus according to any one of the preceding two claims, **characterised in that** the activating element (12) and the blocking slider (25) cannot be moved in and counter to the advancing direction, relative to each other.

12. The administering apparatus according to any one of the preceding three claims, **characterised in that** the blocking slider (25) comprises at least one indentation recess (29) which only overlaps a latching means (20), which forms one of the latching elements (3a; 21), in the releasing position of the drive element (12; 32), such that the latching means (20) can be moved at least partially into the indentation recess (29), in order to release the latching engagement.

13. The administering apparatus according to any one of the preceding claims, **characterised in that** the administering apparatus comprises a dosage setting member (9; 39) which is in dosing engagement with the piston rod (4), such that it is moved relative to the piston rod (4) in a dosing movement for selecting a product dosage, and moved jointly with the piston rod (4) in the advancing direction relative to the front casing section (1, 3) in a delivery movement for delivering the product.

14. The administering apparatus according to any one of the preceding claims, **characterised in that** the administering apparatus comprises a dosage setting member (9; 39) which engages with the piston rod (4) such that it can be moved counter to the advancing direction relative to the front casing section (1, 3) and the piston rod (4) for selecting a product dosage and can be moved jointly with the piston rod (4) in the advancing direction relative to the front casing section (1, 3) for delivering the product dosage.

15. The administering apparatus according to any one of the preceding two claims, **characterised in that** the dosage setting member (9; 39) abuts against a delivery stopper (3c) of the front casing section (1, 3) in the advancing direction, said delivery stopper (3c) limiting the delivery movement in the advancing direction.

16. The administering apparatus according to any one of the preceding three claims, **characterised in that** the drive element (12; 32) pushes against the dosage setting member (9; 39) during its delivery movement and thus slaves the piston rod (4).

17. The administering apparatus according to any one of the preceding claims, **characterised in that** the drive element (12; 32) is mounted by the rear casing section (11) such that it can also perform a dosing movement, and is coupled to the piston rod (4) such that it slaves the piston rod (4) during the dosing movement.

18. The administering apparatus according to any one of the preceding claims, **characterised in that** a blocking means (8; 38) is provided which is engaged with the piston rod (4) in order to prevent the piston rod (4) from moving counter to the advancing direction.

19. The administering apparatus according to any one of the preceding claims, **characterised in that** a blocking means (8) is provided which is in a braking engagement with the piston rod (4) which makes it more difficult for the piston rod (4) to move in the advancing direction.

20. The injection apparatus according to any one of the preceding claims, **characterised in that** the front casing section (1, 3) comprises a reservoir part (1) containing the reservoir (2) and a mechanism holder (3) which is connected to the reservoir part (1) such that it cannot be moved with respect to the advancing direction, and which holds the piston rod (4) and comprises the first latching element (3a).

21. The administering apparatus according to any one of the preceding claims, **characterised in that** a cannula of at most 30 gauge, preferably a 31 or 32 gauge cannula, or a cannula outside the size specified in ISO 9626 having an outer diameter of a 30 gauge cannula at most, forms an infusing part of the administering apparatus.

22. A drive device for the administering apparatus according to claim 1, which can be detachably connected to a reservoir module (10) of the administering apparatus, wherein the reservoir module (10) comprises the reservoir (2) for a product which can be delivered and the piston accommodated in the reservoir (2) such that it can shift in an advancing direction in order to deliver product, said drive device (30) comprising:
a) the rear casing section (11) of the administering apparatus which can be connected to the reservoir module (10), and the latching element (21) for establishing a latching engagement with the reservoir module (10);
b) the drive element (12; 32) which is mounted by the rear casing section (11) such that it is movable;
c) and the blocking slider (25) which acts on the latching element (20) and is coupled to the drive element (12; 32) such that the drive device (30) can only be connected to the reservoir module (10) when the drive element (12) assumes a releasing position with respect to the advancing direction, relative to the rear casing section (11).

23. The drive device according to the preceding claim, **characterised in that** the rear casing section and the second latching element according to any one of claims 2 to 20 form the casing section (11) and the latching element (21) of the drive device (30), and wherein the drive element (12; 32) and the latching block (25) of the drive device (30) are also formed like the drive element and the latching block according to any one of claims 2 to 20.

24. A reservoir module for an administering apparatus, wherein the reservoir module can be detachably connected to a drive device of the administering apparatus, said reservoir module (10) comprising:
a) a front casing section (1, 3) of the administering apparatus, comprising a reservoir (2) for a product which can be delivered;
b) a piston which is accommodated in the reservoir (2) such that it can be shifted in an advancing direction towards a reservoir outlet, in order to deliver product; and
c) a latching element (3a) which is formed by the front casing section (1, 3) for establishing a latching engagement with the drive device (30) or is movably connected to the front casing section (1, 3) for establishing and releasing the latching engagement,
**characterised by**
d) a piston rod (4) which is mounted by the front casing section (1, 3).

25. The reservoir module according to the preceding claim, **characterised in that** the front casing section (1, 3) comprises a radial protrusion on a rear surface region facing away from the reservoir outlet, said radial protrusion forming or forming a part of the latching element (3a).

26. The reservoir module according to the preceding claim, **characterised in that** the front casing section (1, 3) holds the piston rod (4) according to any one of claims 1 to 20.

27. The reservoir module according to any one of the preceding three claims, **characterised in that** the front casing section (1, 3) forms or holds a blocking means (8; 38) according to any one of claims 1 to 20.

28. The reservoir module according to any one of the preceding four claims, **characterised in that** the front casing section (1, 3) forms the delivery stopper (3c) according to claim 15.

29. The reservoir module according to any one of the preceding five claims, **characterised in that** the front casing section (1, 3) forms a guide, preferably a linear guide, for the dosage setting member (9; 39) according to any one of the preceding claims.

## Revendications

1. Appareil d'administration, comprenant :
a) un tronçon de boîtier antérieur (1, 3) avec un réservoir (2) pour un produit capable de se déverser et un premier élément de verrouillage (3a),
b) un piston, qui est reçu avec possibilité de déplacement dans le réservoir (2) dans une direction d'avance vers une sortie du réservoir, afin de faire déverser le produit,
c) une tige de piston (4),
d) un tronçon de boîtier postérieur (11), qui est relié de façon détachable au tronçon de boîtier antérieur (1, 3) et qui comporte un second élément de verrouillage (21) qui se trouve en engagement de verrouillage avec le premier élément de verrouillage (3a), et
e) un élément d'entraînement (12 ; 32) monté déplaçable par le tronçon de boîtier postérieur (11) dans la direction d'avance et à l'encontre de la direction d'avance, ledit élément d'entraînement agissant sur la tige de piston (4) lors d'un mouvement de déversement dans la direction d'avance, afin de déplacer le piston dans la direction d'avance,
**caractérisé par** :
f) un blocage de verrouillage (25) pour les éléments de verrouillage (3a, 21), qui est accouplé à l'élément d'entraînement (12 ; 32) de telle manière que l'engagement de verrouillage ne peut être annulé que dans une position de libération de l'élément d'entraînement (12 ; 32).

2. Appareil d'administration selon la revendication 1, **caractérisé en ce que** l'un au moins des éléments de verrouillage (3a ; 21) est déplaçable dans une direction transversale à la direction d'avance avec son tronçon de boîtier associé (1,3; 11), afin d'établir et d'annuler l'engagement de verrouillage.

3. Appareil d'administration selon la revendication précédente, **caractérisé en ce que** ledit au moins un élément de verrouillage déplaçable (3a ; 21) est déplaçable dans une direction radiale par rapport à un axe longitudinal (L) de l'appareil.

4. Appareil d'administration selon l'une des deux revendications précédentes, **caractérisé en ce que** ledit au moins un élément de verrouillage déplaçable (3a ; 21) doit être actionné pour annuler l'engagement de verrouillage dans la position de libération de l'élément d'entraînement (12 ; 32).

5. Appareil d'administration selon la revendication précédente, **caractérisé en ce que** ledit au moins un élément de verrouillage déplaçable (3 a ; 21) est relié à un bouton de déverrouillage (22).

6. Appareil d'administration selon l'une quelconque des revendications précédentes, **caractérisé en ce que** le tronçon de boîtier antérieur (1, 3) et le tronçon de boîtier postérieur (11) sont immobilisés l'un par rapport à l'autre dans la direction d'avance et à l'encontre de la direction d'avance par les éléments de verrouillage (3a ; 21) qui se trouvent en engagement de verrouillage.

7. Appareil d'administration selon l'une quelconque des revendications précédentes, **caractérisé en ce que** le premier élément de verrouillage (3a) est un composant fixe du tronçon de boîtier antérieur (1, 3), ou **en ce que** le second élément de verrouillage est un composant fixe du tronçon de boîtier postérieur (11).

8. Appareil d'administration selon l'une quelconque des revendications précédentes, **caractérisé en ce que** l'un des éléments de verrouillage (3a ; 21) est agencé sur son tronçon de boîtier associé (1, 3 ; 11) déplaçable vers l'engagement de verrouillage et hors de celui-ci, et un dispositif de rappel (24) agit élastiquement sur l'élément de verrouillage (21) agencé déplaçable afin d'empêcher une annulation autonome de l'engagement de verrouillage.

9. Appareil d'administration selon l'une quelconque des revendications précédentes, **caractérisé en ce qu'**un tiroir de blocage, qui forme conjointement ou à lui seul le blocage de verrouillage (25), et l'élément d'entraînement (12 ; 32) sont accouplés de telle façon que l'élément d'entraînement (12 ; 32) entraîne le tiroir de blocage (25) lors du mouvement de déversement.

10. Appareil d'administration selon la revendication précédente, **caractérisé en ce** tiroir de blocage (25) est déplaçable transversalement à une direction dans laquelle l'un des éléments de verrouillage (3a ; 21) doit être déplacé pour annuler l'engagement de verrouillage.

11. Appareil d'administration selon l'une des deux revendications précédentes, **caractérisé ce que** l'élément d'actionnement (12) et le tiroir de blocage (25) ne sont déplaçables l'un par rapport à l'autre ni dans la direction d'avance, ni à l'encontre de celle-ci.

12. Appareil d'administration selon l'une des trois revendications précédentes, **caractérisé en ce que** le tiroir de blocage (25) comporte au moins un évidement d'enclenchement (29) qui se trouve, uniquement dans la position de libération de l'élément d'entraînement (12 ; 32), en recouvrement avec un moyen de verrouillage (20) qui forme l'un des éléments de verrouillage (3a ; 21), de telle façon que le moyen de verrouillage (20) est déplaçable au moins partiellement jusque dans l'évidement d'enclenchement (29) pour annuler l'engagement de verrouillage.

13. Appareil d'administration selon l'une quelconque des revendications précédentes, **caractérisé en ce que** l'appareil d'administration comprend un organe de réglage de dose (9 ; 39) qui est en engagement de dosage avec la tige de piston (4) de telle sorte que, lors d'un mouvement de dosage pour choisir une dose de produit, il est déplacé par rapport à la tige de piston (4) et, lors d'un mouvement de déversement pour un déversement du produit, il est déplacé conjointement avec la tige de piston (4) dans la direction d'avance par rapport au tronçon de boîtier antérieur (1, 3).

14. Appareil d'administration selon l'une quelconque des revendications précédentes, **caractérisé en ce que** l'appareil d'administration comprend un organe de réglage de dose (9 ; 39) qui se trouve dans un engagement avec la tige de piston (4) tel que pour une sélection d'une dose de produit il est déplaçable par rapport au tronçon de boîtier antérieur (1, 3) et la tige de piston (4) est déplaçable à l'encontre de la direction d'avance, et pour un déversement de la dose de produit, il est déplaçable conjointement avec la tige de piston (4) dans la direction d'avance par rapport au tronçon de boîtier antérieur (1, 3).

15. Appareil d'administration selon l'une des deux revendications précédentes, **caractérisé en ce que** l'organe de réglage de dose (9 ; 39) bute dans la direction d'avance contre une butée de déversement (3c) du tronçon de boîtier antérieur (1, 3) qui limite le mouvement de déversement dans la direction d'avance.

16. Appareil d'administration selon l'une des trois revendications précédentes, **caractérisé en ce que** l'élément d'entraînement (12 ; 32) pousse contre l'organe de réglage de dose (9 ; 39) lors de son mouvement de déversement, et entraîne de ce fait la tige de piston (4).

17. Appareil d'administration selon l'une quelconque des revendications précédentes, **caractérisé en ce que** l'élément d'entraînement (12 ; 32) est porté par le tronçon de boîtier postérieur (11) de façon déplaçable également pour exécuter un mouvement de dosage, et est couplé à la tige de piston (4) de telle façon qu'il entraîne la tige de piston (4) lors du mouvement de dosage.

18. Appareil d'administration selon l'une quelconque des revendications précédentes, **caractérisé en ce qu'**il est prévu un système de blocage (8 ; 38) qui est en engagement avec la tige de piston (4) pour empêcher un mouvement de la tige de piston (4) à l'encontre de la direction d'avance.

19. Appareil d'administration selon l'une quelconque des revendications précédentes, **caractérisé en ce qu'**il est prévu un système de blocage (8) qui se trouve en engagement de freinage avec la tige de piston (4) pour rendre difficile le mouvement de la tige de piston (4) dans la direction d'avance.

20. Appareil d'administration selon l'une quelconque des revendications précédentes, **caractérisé en ce que** le tronçon de boîtier antérieur (1, 3) comprend une partie réservoir (1) contenant le réservoir (2) et un porte-mécanisme (3) qui n'est pas relié en déplacement avec la partie réservoir (1) par référence à la direction d'avance, qui tient la tige de piston (4), et qui comprend le premier élément de verrouillage (3a).

21. Appareil d'administration selon l'une quelconque des revendications précédentes, **caractérisé en ce qu'**une canule au calibre maximum de 30, de préférence une canule au calibre 31 ou 32, ou bien une canule à l'extérieur des dimensions spécifiées dans la norme ISO 9626 avec un diamètre extérieur qui est au maximum celui d'une canule du calibre 30, forme une partie d'injection de l'appareil d'administration.

22. Dispositif d'entraînement pour l'appareil d'administration selon la revendication 1, qui peut être relié de façon détachable à un module réservoir (10) de l'appareil d'administration, dans lequel le module réservoir (10) comprend le réservoir (2) pour un produit susceptible d'être déversé, et le piston reçu dans le réservoir (2) est déplaçable dans une direction d'avance pour le déversement du produit, le dispositif d'entraînement (30) comprenant :
a) le tronçon de boîtier postérieur (11) de l'appareil d'administration, qui est susceptible d'être relié au module réservoir (10) et qui comprend l'élément de verrouillage (21) pour établir un engagement de verrouillage avec le module réservoir (10),
b) l'élément d'entraînement (12 ; 32) monté déplaçable par le tronçon de boîtier postérieur (11),
c) et le blocage de verrouillage (25), qui agit sur l'élément de verrouillage (20) et qui est couplé à l'élément d'entraînement (12 ; 32) de telle façon que le dispositif d'entraînement (30) ne peut être relié au module réservoir (10) que si l'élément d'entraînement (12) occupe par rapport au tronçon de boîtier postérieur (11) une position de libération par référence à la direction d'avance.

23. Dispositif d'entraînement selon la revendication précédente, **caractérisé en ce que** le tronçon de boîtier postérieur et le second élément de verrouillage selon l'une quelconque des revendications 2 à 20 forment le tronçon de boîtier (11) et l'élément de verrouillage (21) du dispositif d'entraînement (30), et **en ce que** l'élément d'entraînement (12 ; 32) et le blocage de verrouillage (25) du dispositif d'entraînement (30) sont réalisés, tout comme l'élément d'entraînement et le blocage de verrouillage, selon l'une quelconque des revendications 2 à 20.

24. Module réservoir pour un appareil d'administration, dans lequel le module réservoir peut être relié de manière détachable avec un dispositif d'entraînement de l'appareil d'administration, le module réservoir (10) comprenant :
a) un tronçon de boîtier antérieur (1, 3) de l'appareil d'administration avec un réservoir (2) pour un produit susceptible d'être déversé,
b) un piston, qui est reçu dans le réservoir (2) déplaçable dans une direction d'avance vers une sortie de réservoir, afin de déverser le produit, et
c) un élément de verrouillage (3a) qui, pour établir un engagement de verrouillage avec le dispositif d'entraînement (30), est formé par le tronçon de boîtier antérieur (1, 3), ou qui, pour établir et pour annuler l'engagement de verrouillage, est relié de façon déplaçable avec le tronçon de boîtier antérieur (1, 3),
**caractérisé par**
d) une tige de piston (4), qui est portée par le tronçon de boîtier antérieur (1, 3).

25. Module réservoir selon la revendication précédente, **caractérisé en ce que** le tronçon de boîtier antérieur (1, 3) comprend, sur une zone enveloppe postérieure détournée de la sortie de réservoir, une saillie radiale qui forme à elle seule ou conjointement l'élément de verrouillage (3a).

26. Module réservoir selon la revendication précédente, **caractérisé en ce que** le tronçon de boîtier antérieur (1, 3) tient la tige de piston (4) selon l'une quelconque des revendications 1 à 20.

27. Module réservoir selon l'une des trois revendications précédentes, **caractérisé en ce que** le tronçon de boîtier antérieur (1, 3) forme ou tient un système de blocage (8 ; 38) selon l'une quelconque des revendications 1 à 20.

28. Module réservoir selon l'une quelconque des quatre revendications précédentes, **caractérisé en ce que** le tronçon de boîtier antérieur (1, 3) forme la butée de déversement (3c) selon la revendication 15.

29. Module réservoir selon l'une quelconque des cinq revendications précédentes, **caractérisé en ce que** le tronçon de boîtier antérieur (1, 3) forme un guidage, de préférence un guidage linéaire, pour l'organe de réglage de dose (9 ; 39) selon l'une quelconque des revendications précédentes.
